# EUROPEAN PATENT SPECIFICATION

(11) **EP 1 800 133 B1**
(45) Date of publication and mention of the grant of the patent: **12.01.2011**
(21) Application number: 05791017.6
(22) Date of filing: 07.10.2005
(51) Int. Cl.: G01N 33/68

(54) **METHODS FOR ANTIBODY LIBRARY SCREENING**
METHODEN ZUM SCREENING VON ANTIKÖRPER-BIBLIOTHEKEN
PROCEDES DE CRIBLAGE DE BANQUES D'ANTICORPS

(30) Priority: 08.10.2004 GB 0422431
(43) Date of publication of application: 27.06.2007
(73) Proprietor: Affitech Research AS, 0349 Oslo (NO)
(72) Inventor: STASSAR, Marike, Josée, Janneke, Gertrud, N-1353 Baerums Verk (NO); REIERSEN, Herald, N-1412 Sofiemyr (NO)
(74) Representative: Webber, Philip Michael
(86) International application number: PCT/GB2005/003866
(87) International publication number: WO 2006/038022

(56) References cited:
- WO-A-02/20822
- STASSAR M J J G ET AL: "CBAS: A new method for the identification of single chain antibodies specific for cell surface antigens" HUMAN ANTIBODIES, vol. 13, no. 1-2, 2004, pages 35-36, XP009058189 & 11TH INTERNATIONAL CONFERENCE ON HUMAN ANTIBODIES AND HYBRIDOMAS; DUBLIN, IRELAND; OCTOBER 06-08, 2004 ISSN: 1093-2607

## Description

This invention relates to a method of screening a phage display library, to identify or select one or more members thereof which are candidate binding partners for one or more target entities, wherein said target entity is a cell surface molecule or a molecule which is attached to a solid phase. The invention further relates to an improved method of separating candidate binding partners for said target entities from other library members.

Phage antibody display technology has proven to be a very suitable technique for the screening and selection of antibodies to defined target antigens (for a review see Hoogenboom et al., 1998, Immunotechnol., 4:1-20). Most strategies have relied on the availability of purified and/or recombinant antigens, which have been screened after immobilization onto a solid support.

Probing the diversity of cell surfaces and identifying binding partners for cell surface molecules specific for certain cell types or disease-related states is the most promising approach for the development of targeted therapies. However, the identification of such binding partners are often challenging experiments.

Conventional methods for the identification of binding partners for cell surface molecules are based on the incubation of cells in suspension or culture with complex phage display single chain Fv (scFv) libraries followed by several washings (usually 5 or more) and acid or alkaline elution of bound phages (see for example Hoogenboom et al., 1999, Eur. J. Biochem., 260: 774-784; Ridgeway et al., 1999, Cancer Research, 59: 2718-2723; Wong et al., 2001, Cancer Immunol. Immunother., 50: 93-101). This is often preceded by removing irrelevant binding partners by negative panning on irrelevant cell types. A more recently developed and more high-throughput method is the screening of cells with a phage display library after immobilization of the cells onto nitrocellulose membranes (Radosevic et al., 2003, J. Immunol. Methods, 272:219-233).

An alternative and time saving approach to the conventional approach for the screening, selection and sorting of cell surface binding peptides using a phage display library has been described by Giordano *et al.,* and is termed the BRASIL method (Biopanning and Rapid Analysis of Selective Interactive Ligands, 2001, Nature Med., 11: 1249-1253). This method is based on a single step organic phase separation of free and cell bound phages which has the advantage that the washing steps essential to the conventional method (i.e. steps in which free phage are washed away from the cells), which are labor intensive, inefficient and result in cells and potential ligands being lost, are avoided.

Surprisingly, it has now been found that, contrary to the teaching of the BRASIL method, a significantly improved method for the screening, selection and identification of binding partners for cell surface molecules, involves subjecting the cells to at least one washing step before organic phase separation. The described method also shows significant improvements over the conventional washing and elution method described above.

At its most general, the present invention therefore provides a method of screening a library of molecules to identify or select one or more members thereof which are candidate binding partners for one or more target entities comprising:
(a) contacting an expression library with one or more target entities;
(b) subjecting said target entities to at least one washing step;
(c) separating target entities which have become bound to one or more members of the expression library from unbound members of the expression library by separation through an organic phase, thereby separating candidate binding partners for said target entities from other library members, wherein said expression library is a phage display library and wherein said target entity is a cell surface molecule or a molecule which is attached to a solid phase.

Viewed alternatively, the present invention provides an improved method of separating from an expression library candidate binding partners which have become bound to a target entity from other non-bound library members comprising steps (a) to (c) as defined herein, wherein said expression library is a phage display library and wherein said target entity is a cell surface molecule or a molecule which is attached to a solid phase.

The library of molecules to be screened in accordance with the present invention is a phage display library protein expression library. Examples of expression libraries are well known and described in the art and include display libraries such as phage display libraries (for example Winter et al., WO90/05144; McCafferty et al., WO92/01047), bacteria (for example Samuelson et al., 2002, J. Biotechnol. 96, 129-154), covalent or non-covalent display libraries such as ribosome display libraries (for example as published in WO92/02536 of The Regents of the University of Colorado, WO93/03172 of University Research Corporation and WO91/05058 of Kawasaki), or PROfusion libraries (Phylos, Inc.), wherein the expressed protein is bound to the mRNA encoding it, or covalent or non-covalent display systems whereby the expressed protein is bound to the DNA encoding it (for example via a covalent linkage as in the systems described in WO98/371 of Actinova Ltd or via cis acting proteins in the cis-display systems as described in WO04/022746), or yeast display systems (for example those described by Wittrup, K. D. et al., WO 99/36569; Wittrup, K. D. (2001) Curr. Opin. Biotechnol. 12, 395-399; Lee, S. Y. et al (2003) Trends in Biotechnol. 21, 45-52), or bacterial two hybrid systems (e.g. Chien et al., 1991, Proc. Natl. Acad. Sci. USA 88, 9578). Alternative types of expression library such as bacterial expression libraries or other libraries wherein proteins are expressed and secreted as soluble fragments are known. Chemical libraries can also be screened, e.g. synthetic peptide libraries. Libraries for use in the present invention are phage display libraries.

The proteins expressed by the expression library can be of any appropriate length providing that said length is sufficient to enable a protein to act (or potentially act) as a binding partner for a target entity. Thus, said proteins may be short linear peptides (e.g. of the order of 5-50 or 7-30 amino acids in length), or longer peptides or polypeptides which may be folded rather than linear. In contrast to the prior art BRASIL method as described in Giordano et al., *supra,* the methods of the present invention have advantageously been shown to be useful to screen expression libraries which comprise longer polypeptides, more specifically antibody expression libraries, as opposed to short peptide libraries.

Thus, the proteins of the expression library may be encoded for by whole genes or fragments thereof, e.g. the nucleic acids encoding the expression library members may be a cDNA or mRNA library or fragments thereof, for example generated from a particular cell type or may be a genomic DNA library or fragments thereof.

Preferred expression libraries express polypeptide binding partners which are candidate ligands, receptors, enzymes, substrates, antigens etc., or fragments thereof, and especially preferred phage display expression libraries express antibody molecules or antibody fragments which can then be screened for candidates which bind to one or more known or unknown target antigens. Said antibody expression libraries may express antibodies in any appropriate form and may comprise whole antibody molecules or antibody fragments such single chain antibodies (e.g. scFv), Fab, Fv, Fab'2, diabodies, bispecific antibodies, minibodies, heavy chains or light chains, cameloid antibodies, tetrabodies, single domain antibodies, triabodies, etc. A preferred format of antibody fragments are scFv fragments.

The antibody molecules or fragments may be of any Ig isotype, such as IgG, IgM or IgA and so forth, and the expression libraries may comprise antibodies of one or more of these subtypes.

Many antibody libraries are known and described in the art and any of these may be screened using the methods of the invention.

When an expression library is referred to herein, the term can be used to refer to such a library at the nucleic acid or protein level, i.e. before or after expression of the encoded proteins has taken place. Clearly however, such expression libraries must be present at the protein level in order for interaction with the target entities to take place.

Methods for constructing such expression libraries and nucleic acids encoding them are well known and described in the art and any known expression library or newly developed expression library can be used in this regard. For example such libraries may be comprised of naturally occurring polypeptides or fragments thereof or may be wholly or partially random or synthetic. For example, in the case of antibody expression libraries, the libraries may be derived by cloning nucleic acids from a naive population of lymphocytes from a healthy donor (e.g. as described in EP-A-368684), or from an enriched population of lymphocytes, e.g. lymphocytes derived from a patient which has been exposed to antigen, or immunized with a vaccine or for example tumour cells (e.g. as described in WO03/095491 of Affitech AS), or from lymphocyte populations which have been enriched by panning on particular antigens.

The expression libraries and in particular the antibody expression libraries may be derived from any appropriate source, preferably from a mammalian source, more preferably a human source. Chimeric expression libraries or humanized expression libraries may also be used. The libraries may also be created by choosing a naturally occurring scaffold and including randomized sequences at appropriate places. Alternatively, the scaffold may be based on one or more consensus sequences derived from a variety of naturally occurring frameworks.

Generally, the techniques used to prepare the library constructs will be based on known genetic engineering techniques. In this regard, nucleic acid sequences encoding the actual protein or peptide which is to be expressed in the expression library and which generally vary between different library members, thereby providing the library diversity, are incorporated into expression vectors appropriate for the phage display type of expression system to be used. Appropriate expression vectors for use in phage display are well known and described in the art (see for example the expression library documents discussed above). Phage display is the expression library of choice, so either phage or phagemid vectors may be used, although phagemid vectors are preferred.

Once generated the nucleic acid molecules encoding for different library members (i.e. encoding the peptides which vary between the library members) can also be further diversified before screening, using standard techniques, for example by mutation involving the addition, deletion and/or substitution of one or more nucleotides in a controlled (e.g. site directed mutagenesis) or random manner, or by domain swapping, cassette mutagenesis, chain shuffling etc. Synthetic nucleotides may be used in the generation of the diverse nucleic acid sequences. Thus, all or part of the nucleic acids encoding the expression peptides can be synthesised chemically or be derived from various organisms or cell types.

The library constructs may optionally additionally contain other appropriate components, for example origins of replication, inducible or non-inducible promoters for initiating transcription, enhancers, antibiotic resistance genes and markers, general tags or reporter molecules, primer binding sites to enable amplification of the constructs by e.g. PCR, or other desirable sequence elements. Appropriate sources and positioning of such additional components within the library constructs so that they perform their desired function would be well within the normal practice of a skilled person in the art.

The inclusion of markers or reporter molecules can be particularly useful in the expression libraries which are used in the present invention. Such markers or reporter molecules may be directly or indirectly detectable and include for example short sequence tags which can be recognised by an antibody, radiolabels, fluorescent labels or labels which can be detected enzymatically. Other markers which can be used include one partner of a binding pair such as streptavidin:biotin. Such markers are typically general markers which are present in all the library constructs and can be used to detect the presence of the library members. In addition, the strength of signal detected can also be used to quantitate the amount of a particular library member present. Such quantitation of the amount of a library member present can prove extremely useful in determining the affinity of a target entity for a library member. Alternatively or additionally, if for example one or more of the nucleic acids making up the library molecules are labelled with different labels, or immobilized onto differently sized or differently labelled beads, information about the content, e.g. the sequence of the nucleic acid molecules can be obtained.

In embodiments of the invention where the methods of the invention are combined with the AffiSelect method (see below) then the inclusion of general tags or markers in the library constructs and in the expressed library members (candidate binding partners) is particularly important and such tags are used to facilitate the binding of library members (candidate binding partners) to a solid phase. Appropriate tags and markers are described below in the description of the AffiSelect method.

The term "target entity" as used herein, refers to an entity or molecule of interest to which it is desired to identify a binding partner from an expression library, and which is attached to or in some way associated with a moiety which can be subjected to separation through an organic phase, e.g. a moiety which will progress through and collect at the bottom of an organic phase when appropriate separation conditions, preferably a centrifugal force, is applied. Such target entities may be known or unknown molecules for which it is desired to identify candidate binding partners. The moiety is a cell or a solid phase.

Such target entities also have to be capable of binding or otherwise interacting with members of the protein expression library being screened. Thus said target entities are entities which are capable of binding to proteins or peptides and can be proteins/peptides, glycopeptides, carbohydrates, lipids, glycolipids, small chemical molecules, nucleic acids, etc., attached to a solid phase or present on the surface of a cell. The solid phase or cell represents an appropriate moiety which can be subjected to separation through an organic phase. Preferred is a particulate solid phase, such as beads. Thus such target entities may be cell surface molecules attached to or being components of whole cells, or membrane fractions of cells or molecules, for example free, naked, isolated or purified molecules, attached to a solid phase, or cell membrane fractions attached to a solid phase. Preferred target entities are proteins or peptides, e.g. antigens. More preferred target entities are cell surface molecules (i.e. molecules present *in situ* on the surface of cells or membrane fractions of cells), and in particular cell surface proteins or peptides, e.g. cell surface antigens.

In embodiments of the invention where the target entity is a molecule attached to the surface of a solid phase then this molecule can be derived from any appropriate source, i.e. can be a naturally occurring protein, carbohydrate, lipid, glycolipid, etc., which has been isolated or purified from its natural surroundings, or can be a recombinant or synthetic molecule, e.g. a recombinant protein or peptide or a synthesised chemical molecule. Isolated, purified or recombinant antigens are particularly preferred target entities in this regard.

The term "cell" as used herein refers to any type of biological particle of interest which can be separated through an organic phase. For example this term includes prokaryotic cells such as bacteria, viruses (in particular aggregated virus particles, e.g. particles aggregated by antibodies or by chemical modification, or coated virus particles with a preference for an organic phase as opposed to an aqueous phase), eukaryotic cells (including lower eukaryotic cells such as yeast cells and higher eukaryotic cells such as mammalian cells, in particular human cells). Biological particles that are not normally dense enough to be separated through an organic phase are generally excluded from the term "cell" as used herein. Fragments of cells are also included within the ambit of this term, e.g. membrane fractions, cell wall fractions, cell wall proteins, membrane proteins, etc., providing these retain or are conferred with the ability to be separated through an organic phase. (For example in the case of cell wall proteins and membrane proteins, these may need to be aggregated in order to facilitate separation through an organic phase).

The cells may be naturally occurring cells or a mixture of cells (e.g. cells derived from a biopsy or some other sample from a mammalian subject), or cell lines (including immortalized cells and genetically engineered cell lines, for example cells transformed or transfected with a nucleic acid encoding a particular target entity or a plurality of cells transformed or transfected with a library of target entities, e.g. a cDNA library, which target entities are then expressed on the surface of the cells), etc. Preferred libraries of target entities are libraries produced from disease associated entities, e.g. disease associated cells or pathological agents such as viruses or bacteria. A particularly preferred library is a tumour cell library or a viral cell library (e.g. c-DNA library) to enable candidate binding partners to tumour or viral associated proteins to be identified. Appropriate eukaryotic cells (and other cell types) for transfection, such as for example COS cells, are well known and described in the art and any of these may be used. Cells which overexpress said target entity can also be used.

Preferred "cells" for use in the present invention are eukaryotic cells or membrane fractions thereof. Especially preferred cells are those which are associated with a disease state such as for example cancer cells or cell lines, for example breast cancer or lung cancer cells or cell lines, or lymphocytes (e.g. peripheral blood lymphocytes) from a diseased patient, or cells infected with a virus and thereby presenting viral proteins on their surface.

The cells to be used in the methods of the invention can be derived from any appropriate source, for example the cells can be derived directly from a mammalian subject or can be obtained from *in vitro* cultures. If an *in vitro* culture is used then said cells may be cultured in suspension or as monolayers and may be used in the methods of the invention in a live or fixed state. It is generally preferred that live cells are used because then the target entity on the surface of the cell is present in its native form which means that the candidate binding partners selected then recognize the target entity in its native form, thereby increasing the chances that such candidate binding partners will be of therapeutic or diagnostic use. It is generally preferred that freshly isolated cells rather than frozen or stored cells are used in the methods of the invention. However, panning on tissue slides and frozen material is also possible.

In embodiments of the invention where a target entity is attached to the surface of a solid phase, any appropriate solid phase may be used providing it can be separated through an organic phase. A solid phase of the appropriate density, size and shape to achieve this can readily be selected by a person skilled in the art. However it can be seen that particulate solid supports would be particularly preferred to achieve this. Particularly preferred supports are beads which may optionally be magnetic or at least magnetisable, for example polymer beads carrying superparamagnetic particles may be used. Such supports are well known and documented in the art and it is within the normal practice of a skilled person to select the most appropriate support for use in the methods of the invention.

Particulate supports, e.g. beads are particularly preferred because they are easy to manipulate *in vitro* and facilitate automation. For example if the beads are magnetic they can be separated from a sample by a magnetic field and then washed. Alternatively if the beads are labelled, for example with a fluorescent tag, the beads can be separated by flow cytometry. Suitable labelled, unlabelled, and magnetic beads are available commercially from Dyno Specialty Polymers AS of Lillestrøm, Norway and Dynal Biotech ASA. Particular examples of magnetic beads which can be used in the methods described herein are the M-450, M-270 or M-280 beads from Dynal Biotech ASA, Norway. Particular examples of non-magnetic beads which can be used in the methods described herein are 5 µm glycidyl methacrylate microbeads (Bangs Laboratories, Carmel, IN). Methods of attaching the biomolecular target entities to solid phases are also well known and described in the art.

"One or more" as used herein in connection with the term "target entity" refers to one or more distinct types of target entity, for example one or more different cell surface molecules or polypeptides. In other words the methods of the invention can be used to screen for candidate binding partners to one target entity or a plurality or library of target entities (e.g. can be used for library vs library screening which is particularly advantageous). If more than one target entity is involved in the screening, these may be attached to or associated with the same or different moieties which can be subjected to separation through an organic phase. For example, it can be seen that if the target entities are cell surface molecules attached to whole cells or membrane fractions thereof, then candidate binding partners for a number of different cell surface molecules are likely to be identified (in other words, this is an example of a case where screening for candidate binding partners to a library of target entities takes place, i.e. is a form of library vs. library screening). Of course, if desired, this procedure can be biased towards the selection of candidate binding partners for a particular cell surface molecule, e.g. in the case of a cell having an immunodominant target entity, or by selecting a cell which was known or had been selected to overexpress the target entity of interest or had been engineered to overexpress such a target entity. Further examples of libraries of target entities are c-DNA libraries or other genetic libraries, transfected and expressed in cells, for example a subtracted tumour c-DNA library transfected and expressed in eukaryotic cells.

Each target entity can of course be present in multiple copies in the screening reaction (indeed, this is generally preferred), e.g. if the target entity is a cell surface molecule then multiple copies of this are preferably present either on the same cell and/or on multiple cells of the same or different type.

In particularly preferred embodiments of the invention, the expression library is a phage display antibody library and the target entities are cell surface molecules (cell surface antigens). Such methods of screening are termed Cell Based Antibody Selection (CBAS).

The step of bringing the target entities into contact with the expression library, or vice versa, can be carried out in any appropriate way under conditions such that appropriate binding partners in the expression library can interact with or bind to the target entities which are present. Such conditions will generally vary depending on the nature of the expression library, the target entity and the moiety with which the target entity is associated. However, appropriate conditions to facilitate binding can be readily determined by a person skilled in the art. Such a "contacting" step will generally occur in solution or in an aqueous medium or in some other context such that the expression library - target entity mixture will be immiscible with the organic phase, as this will facilitate the subsequent organic phase separation step. Thus, if the target entity is present on a cell which is cultured as a monolayer or cells which are obtained from a mammalian subject, e.g. from a mammalian organ or tissue, then these are generally harvested and resuspended in an aqueous medium by appropriate techniques before being contacted by the expression library. However, contacting the expression library with a monolayer of cells is also possible, after which these cells can be harvested and resuspended in an aqueous medium by appropriate methods.

Exemplary "contacting" conditions may comprise incubation on ice or at 4°C for between 30 minutes and 4 hours. Alternatively, carrying out the contacting step at room temperature or 37°C is possible and may be preferable in some cases. The expression library - target entity mixture may optionally be subjected to gentle rocking, mixing or rotation. In addition other appropriate reagents such as blocking agents to reduce non specific binding may be added. For example 1-4% BSA or other suitable blocking agent (e.g. milk) may be used. It will be appreciated however that the contacting conditions can be varied and adapted by a skilled person depending on the aim of the screening method. For example, if the incubation temperature is increased, for example to room temperature, this may increase the possibility of identifying binders to a different subset of target entities, e.g. binders to cell surface proteins which are readily internalized. Again such adaptations to the conditions are within the ambit of the skilled person.

The size and complexity of the expression library to be used in the methods of the present invention may be varied, as may the total number of copies of target entities included in the contacting step. For example, preferably a phage titre in the region of 10⁸-10¹³ is used, more preferably in the region of 10¹¹ phage.

Conveniently the number of copies of target entities can be varied by altering the number of moieties present with which the target entities are associated. For example, the target entity is a cell surface molecule, or a polypeptide etc, attached to a solid support, so the number of target entities can be adjusted by increasing or decreasing the number of cells or solid supports present in the contacting mixture, or by increasing or decreasing the number of polypeptides, etc., attached to an individual solid support. Again, the number of target entities required can readily be determined by trial and error, but for example when the target entity is a cell surface molecule, 10⁵ to 10⁷ cells might conveniently be used.

The appropriate concentration of target entities might also readily be determined by trial and error, but for example, when the target entity is a cell surface molecule a concentration of 1x10⁵ - 2x10⁶ cells/ml might conveniently be used.

The washing steps (b) are carried out after the target entities have been in contact with the expression libraries under the appropriate conditions such that binding/interaction between the target entities and the appropriate library members has occurred, but before organic phase separation. It is important to note that the claimed screening method does not preclude additional washing steps being carried out at other points in the procedure, e.g. before step (a), e.g. in the preparation of the target entities for contact with the expression library, or after step (c) e.g. in the further analysis of the target entities or expression library members. However, methods which do not involve a washing step after the step of contacting the expression library with the target entities and before organic phase separation (e.g. the BRASIL method of Giordano et al., *supra*) are excluded.

The washing steps may be carried out in any appropriate way depending on the nature of the target entity and the moiety to which it is attached. For example, if the target entity is attached to a cell or a membrane fraction of a cell, then conveniently said washes take place by centrifuging the expression library - target entity mixture under conditions such that the cells form a pellet, removing the supernatant, and then resuspending the pellet in an appropriate aqueous medium (for example the same medium as the contacting step was carried out in). Such steps of pelleting cells and resuspension would constitute one wash. Such conditions would generally also be appropriate for the washing of target entities associated with a particulate solid phase. If however the target entities were associated with for example a magnetic solid phase, then the wash steps (b) could conveniently be carried out by applying magnetic field to the vessel in which the contacting step had been carried out, removing the supernatant and resuspending the solid phase in an appropriate aqueous medium. Appropriate methods of washing particulate solid phase are well known person skilled in the art. Again such steps of magnetic separation and resuspension would constitute one washing step.

At least one washing step (b) is carried out in the method of the invention, for example at least 1, at least 2, at least 3, or at least 4 washing steps can be carried out. Preferably less than 5 washing steps are carried out, i.e. preferably 1, 2, 3 or 4 washing steps are carried out in step (b). Most preferably 1 or 2 or 3 and especially . preferably 2 washing steps are carried out in step (b) of the method. This preferred number of washing steps has the additional advantage that the method is still significantly faster and less laborious then the conventional method which involves at least five washing steps.

These washing steps will result in the removal of a proportion of the non-bound expression library members, i.e. the members which have not, or have unspecifically, interacted with a target entity. A further proportion of the non-bound expression library members will then be removed in step (c) of the method, i.e. the organic phase separation step.

To facilitate the organic phase separation, after the washing steps (b) the target entities contained in an appropriate polar medium for example an aqueous medium are brought into contact with an organic phase. Said aqueous medium may be overlayed on top of the organic phase or the organic phase may be laid on top of the aqueous medium. In the former case, when centrifuged, the bound target entities collect and preferably pellet in the organic phase, while the non bound library members remain in the aqueous medium. In the latter case, when centrifuged, the aqueous medium containing unbound expression library members passes upwards through the organic phase whilst the bound target entities collect and preferably pellet beneath the organic phase. Alternatively, it is also possible to mix the two phases, which then separate during centrifugation. Thus, the same result is obtained.

The term "organic phase" as used herein refers to a fluid phase which is non-miscible with water or other polar media or aqueous media. Organic phases suitable for use in the invention are those which, when subjected to appropriate conditions, for example centrifugation, will allow the separation of bound target entity - expression library member complexes from non-bound expression library members, for example allow the bound target entity - expression library member complexes to pass through but will exclude unbound members of the expression library, i.e. will exclude non-complexed or free members of the expression library. Clearly any such experimental system is not 100% perfect. Thus, an appropriate organic phase is one which excludes a majority or a significant proportion of or which substantially excludes unbound members of the expression library.

In addition, an appropriate organic phase is selected such that it causes minimum disruption or damage to the target entities, the moieties to which they are attached, the expression library members and the binding reaction between the target entities and the library members in the mixture. This is particularly the case for the target entities, the expression library members and the binding reaction between the two. For example, an organic phase should be selected so that such components are suitable for use in any appropriate downstream analysis steps once they have been collected from the organic phase. However, in general, in order to minimize any harmful effect the organic phase might have on the biomolecules in the mixture, the time which the biomolecules are in contact with the organic phase is generally kept to a minimum.

Appropriate organic phases for use in the claimed methods can readily be determined by a person skilled in the art based on the above functional requirements. Examples of organic phases which may be used in the claimed methods are phthalate based organic phases such as those discussed in Giordano et al., *supra,* e.g. dibutyl phthalate: cyclohexane (9:1 [v:v]) or dibutyl phthalate: diisooctyl phthalate (4:6 [v:v]), or a water immiscible oil such as Versilube F50 oil (Alfa Chemicals Ltd., Woringham). Thus, a water immiscible oil may be used in order to separate expression library - target entity complexes (and in particular phage - target entity complexes) from unbound expression library members (and in particular unbound phage particles).

The separation through the organic phase is generally carried out by centrifugation at an appropriate speed to pellet or at least collect the target entities which have become bound to one or more members of the expression library (the bound target entities) in the organic phase, while all, or a significant proportion, or the majority of the unbound expression library members remain in the aqueous phase. Appropriate centrifugation conditions to effect this separation will depend on the nature of the target entity (and the moiety to which these are attached), and the particular organic phase used. However, where cells are used and cell surface molecules are target entities then exemplary conditions might be centrifugation at 10000-11000g for 5 to 10 minutes at room temperature or 4 °C.

After the separation step (c) the bound target entities can be collected from the organic phase and optionally subjected to further analysis. Such a collection step can if desired be facilitated by freezing the organic phase and removing the pellet containing the bound target entities for example by cutting the centrifuge tube at the appropriate point. Such a freezing step, followed by a thawing step (at some future point after the bound target entities have been collected), does not generally have an adverse effect on the downstream analysis of the bound target entity - expression library member complexes. However, it is to be recommended that this be checked in suitable experiments before such a step is included in any experimental procedure.

The methods of the present invention may involve further additional steps. For example, the expression library may be pre-panned to remove some non-desired expression library members or reduce the complexity of the library, by contacting the expression library with one or more non-relevant (non-target) entities e.g. non-target biomolecular entities, or cells which are not of interest, before the expression library is brought into contact with the target entities in step (a) of the method. Such pre-panning is also termed "negative panning". For example, the expression library may be pre-panned with cells, or membrane fractions thereof, which are not of interest, e.g. which do not express the desired target entities of interest or express the target entities at a low level. For example, if the method is designed to identify expression library members which bind to a particular type of cancer cell, pre - panning may be carried out by contacting the expression library members with a different or irrelevant cell type, e.g. a different type of tumour cell or a non-tumour cell type such as lymphocytes or endothelial cells or other cells which are not of interest. The aim of said pre-panning steps is to remove a proportion of the expression library members which will not bind the target entities of interest. Said pre-panning steps are additional steps to steps (a), (b) and (c) of the method and thus steps (a), (b) and (c) of the claimed method do not encompass such pre-panning steps. Said pre-panning steps can be carried out by any appropriate method, e.g. by the conventional method or even the BRASIL method described above. However, preferably the pre-panning steps will be carried out using steps (a), (b) and (c) as outlined above, except that in step (a) the expression library is contacted with non-relevant (non-target) entities and it is the non-bound expression library members which should be retained. Thus, the supernatants from any washing steps (b) should be retained rather than discarded and in the separation step (c) the unbound library members which have not been separated through the organic phase are retained. This depleted expression library can then be subjected to the method of the invention.

One or more pre-panning rounds can be carried out on the same or different non-relevant entities.

Steps (a) to (c) of the method of the invention can be regarded as constituting one round of panning of target entities against expression library members. Although after the separation step (c) in the first round of panning, the bound target entities can be collected from the organic phase and optionally subjected to further analysis, generally however, in order to further reduce the complexity of the expression library and obtain a suitably enriched population of candidate binding partners for said target entities to enable a productive and time effective further analysis, one or more further panning rounds can be carried out.

Further panning rounds generally involve taking the bound target entities from step (c) of the method, separating, detaching, eluting or isolating the expression library members from the target entities (optionally expanding or amplifying the expression library members) and subjecting said expression library members to a further round of steps (a) to (c) of the method. The expression library is a phage display library, so it is possible to incubate the target entity - bound phage complexes with bacteria. This will elute the phages from the target entity and infect the bacteria so that the phage particles can be amplified ready for example for use in step (a) of the claimed method.

A skilled person can readily determine the number of rounds of panning, if any, which are required or desired. The expression library is a phage display library, so a simple polyclonal ELISA assay of candidate binding partners from step (c) of the method against the target entity can give an indication of the proportion of positive candidates present and whether a further round of panning is desired. In this regard, an increase in library member (phage) binding to target entities (e.g. provided on cells of interest) but not to non-target entities (e.g. provided by cells not of interest) (used as comparative controls) can be enough to show that sufficient numbers of panning rounds have taken place. As each round of panning (i.e. each repeat of steps (a) to (c) of the method) can result in the loss of desired expression library members the number of panning rounds are generally kept to a minimum. Indeed, this is a further advantage that the improved methods described herein have over the BRASIL method and the conventional method based solely on washing steps, as in comparative tests it has been shown that suitable enrichment can be obtained after fewer rounds of panning. For example, in tests described herein, the improved method of the present invention showed suitable enrichment after round 2 of panning as opposed to round 3 (conventional method) or round 4 (BRASIL).

Thus, steps (a) to (c) of the method can be repeated one or more times, for example up to 4 or 5 times. However, in preferred embodiments of the invention, only 1, 2 or 3 rounds of panning (i.e. 1, 2 or 3 rounds of the steps (a) to (c)) are carried out before the candidate binding partners and /or the target entities are subjected to further analysis.

Comparative tests also show that the modified method described herein can result in the identification of an increased number of binding partners for said target entities, i.e. can result in the identification of an increased number of positive clones. For example, after 3 rounds of panning, 41 % of the candidate binding partners selected by the method of the invention were shown to be positive for a target entity in comparison to 12% for the BRASIL method and 28% for the conventional method.

Thus, viewed alternatively, the present invention provides an improved method for the identification of binding partners to target entities comprising carrying out one or more rounds of steps (a) to (c) as defined herein, wherein said expression library is a phage display library and wherein said target entity is a cell surface molecule or a molecule which is attached to a solid phase.

Of course, it may be that if the expression library which is initially screened contains relatively few members or is for example an enriched library, for example is an antibody library isolated from patients which have been exposed to an immunochallenge as described in WO03/095491, *supra,* then further rounds of panning may not be required. Again, this can be readily ascertained by a person skilled in the art, for example using the methods as described above.

Once any appropriate further panning rounds have been carried out then the bound target entities are collected from the organic phase of step (c) and can be subjected to further analysis or uses. Said further analysis or uses generally require the candidate binding partners to be detached, removed, isolated or eluted from the target entities and preferably the candidate binding partners are expressed or produced in isolation from said target entities. Thus, the methods of the present invention may comprise an optional step (d) wherein said candidate binding partners are detached, removed, eluted, or preferably isolated from said target entities, or are expressed or produced in isolation from said target entities. For example in the case where the expression library is a phage library and the target entities are cell surface molecules, said further analysis or uses generally involves the isolation of candidate binding partners by infection of bacteria and cloning the DNA encoding the candidate binding partner into a suitable expression vector. Such an infection step can also allow the amplification of the candidate binding partners.

Said further analysis may involve a further analysis of either or both the members of the expression library which have bound to the target entities (i.e. the candidate binding partners) or the target entities themselves. Thus, the methods of the invention allow for the screening and identification of both novel expression library members (novel binding partners) and novel target entities, e.g. novel cell surface molecules or proteins such as novel antigens.

Appropriate methods for analyzing the expression library members (candidate binding partners) would be well known to a person skilled in the art. Generally, a first step in the further analysis would involve assays or tests to verify that the selected expression library members (candidate binding partners) did indeed bind to the target entities of interest. Any appropriate assay may be used in this regard. However, in general, as described above, a polyclonal ELISA assay could be used in this regard. As discussed above, this verification step would also provide an indication as to when the selected library members were sufficiently enriched for binders to the target entity of interest such that screening can be stopped and the more detailed analysis of the selected clones can be started.

Thus, once the verification step has been completed, this is generally followed by an analysis of single clones. This analysis can be carried out by standard methods. For example for phage expression libraries such analysis includes cloning the phage presented polypeptides into a soluble format (e.g. cloning phage presented scFv into soluble scFv or Fab format) and using the soluble format polypeptides to carry out ELISA on cells (Hoogenboom et al., 1999, *supra*) or purified antigens (using soluble fragments), filter screening assays (Radosevic et al., *supra*), FACS (Ridgway et al., *supra*), Guava assays (Gillis and Fishwild, Application note: Monoclonal Antibody Specificity using the Guava CellPaint Assay, Guava Technologies, Inc., 2004) or immunofluorescence assays (Wong et al., *supra*), staining tissue slides or cells and other immunohistochemistry methods. All these methods are well established in the literature and one or more of them may be used to analyse the clones. Alternatively, direct screening methods such as those described in WO03/095491, or an FMAT analyser (8200 Detection system - Applied Biosystems) can be used for further characterisation of the clones. Finally, a method termed the AffiSelect method can be used to further analyse the candidate binding partners. Details of this method are discussed in a separate section below.

Thus, said further analysis of the candidate binding partners, preferably involves the expression or production of the candidate binding partners, preferably in a soluble format, and assaying or testing the binding activity of the candidates to the target entity of interest. Any appropriate binding assays can be used. However, preferred assays are one or more of ELISA, filter screening assays; FACS, immunofluorescence assays, immunohistochemical assays, Guava assays, 8200 cellular detection assays or AffiSelect assays (see below).

As a negative control, the candidate binding partners are conveniently also screened against non-target entities, e.g. irrelevant cell types or cell types that do not express the target entity on the surface or express the target entity only at low levels, or irrelevant naked or purified molecules, as appropriate.

In all these methods (except the AffiSelect method, which is described in detail below) detection of bound candidate binding partners is facilitated by using reagents which recognize some kind of tag or label on the expression library member. The expression library is a phage library, so detection can for example occur via the use of an antibody to a phage coat protein, e.g. an anti-Fd antibody. Appropriate tagging and detection systems are well known and described in the art.

At any stage the diversity of the candidate binding partners can be tested by restriction digestion of the encoding DNA followed by sequencing or PAGE analysis. Again such methods are well known and described in the art (Hoogenboom *et al., supra,* Ridgway *et al., supra*).

In the embodiments of the invention where the target entity is an unknown molecule, e.g. an unknown cell surface molecule, in particular an unknown tumour surface molecule, then the nature of this can also readily be analyzed and the target entity identified using a specific binding partner identified from the expression library as a tool. This is particularly convenient when the binding partner is an antibody molecule, for example an scFv molecule. For example, as discussed above, such binding partners from the expression library are generally engineered to contain a tag or label which can facilitate detection and can be used for example to analyze the tissue distribution of the unknown molecule, e.g. by binding to tissue sections (Pereira et al., J. Immunol. Methods, 1997, 203:11-24). Alternatively, the binding partner can be immobilized on a solid phase and used to purify the unknown molecule, e.g. by affinity chromatography. Once purified then the identity of the unknown molecule could be ascertained by appropriate tests dependant on the nature of the molecule. For example, if the unknown molecule is a protein, the purified protein could then be identified and its encoding DNA sequence determined by peptide sequencing and cloning using methods well known and described in the art. Alternatively, DNA encoding the unknown protein might readily be determined by screening a cDNA library with the identified binding partner. A highly diverse cDNA library from an appropriate source could be used in this regard. However, preferably, a smaller, restricted cDNA library would be used. For example, if the unknown protein was expressed on the surface of a particular cell type, then a cDNA library (e.g. a cDNA display library such as a phage display library) could conveniently be made from these cells and screened with the binding partner (see for example Ridgway *et al., supra*), for example by panning the display library on solid supports to which the identified binding partner is attached. In addition, if the binding partner identified from the expression library is an antibody then other antibody based assays such as immunoprecipitation, Western Blot, expression profiling or immunohistochemistry could be carried out in order to isolate or characterise the target entity.

Thus, it can be seen that not only can the methods of the invention be used to select and identify binding partners to target entities from appropriate expression libraries but they can also be used to identify novel and unknown target entities. This is important as it may lead to the identification of novel cellular targets for therapy or diagnosis, as well as novel agents with potential for use in therapy or diagnosis, i.e. the binding partners.

Thus, the present invention also provides a method for isolating and/or identifying an unknown target entity comprising steps (a) to (c) (and optionally additional steps) as defined herein, (d) isolating one or more expression library members which bind to said unknown target entity and (e) using said library member to isolate and/or identify the target entity to which it binds wherein said expression library is a phage display library and wherein said target entity is a cell surface molecule or a molecule which is attached to a solid phase.

Methods of the invention can thus be used to select, identify or isolate binding partners for a target entity, or a novel target entity *per se,* which can then be isolated, produced or manufactured for various downstream uses. As such, binding partners/proteins or target entities identified or selected using the methods of the invention are disclosed herein. Thus, a further aspect of the present invention provides a method of selecting, identifying and/or isolating a library member which is a specific binding partner for a target entity, or a method of selecting, identifying and/or isolating a target entity *per se*, from an expression library, said method comprising the steps of screening an expression library using steps (a) to (c) (and optionally additional steps) as defined above to select molecules which display certain properties and optionally (e) identifying and/or isolating the relevant library member (s) which are specific binding partners for the target entity and optionally (f) using said library members to identify the target entity to which it binds wherein said expression library is a phage display library and wherein said target entity is a cell surface molecule or a molecule which is attached to a solid phase.

Once appropriate nucleic acid fragments encoding binding partners or target entities with particular properties have been identified, the nucleic acids encoding the polypeptides can, if desired, be subjected to affinity maturation, for example to try and identify binding partners with further improved properties. Such affinity maturation can be performed by carrying out any conventional form of mutagenesis, including but not limited to the addition, deletion and/or substitution of one or more nucleotides in a controlled (e.g. site directed mutagenesis) or random manner, error-prone PCR, domain swapping, cassette mutagenesis and chain shuffling, etc., prior to repetition of the screening cycle. Such affinity maturation can if desired be performed after the verification steps described above and before the single clone analysis takes place.

When one or more binding partners or target entities have been selected, identified, isolated and/or purified using the methods of the invention, these candidates, or a component, fragment, variant, or derivative thereof may be manufactured and if desired formulated with at least one pharmaceutically acceptable carrier or excipient. Alternatively, these molecules may take the form of nucleic acids encoding said protein molecules, which nucleic acids may in turn be incorporated into an appropriate expression vector and/or be contained in a suitable host cell. Thus, nucleic acid molecules encoding said binding partners or target entities, or expression vectors containing said nucleic acid molecules are disclosed herein.

Once a particular binding partner or target entity, or a component, fragment, variant, or derivative thereof, has been selected, identified, etc., in accordance with the present invention, the expression vector encoding the selected binding partner or target entity can readily be used (or adapted for use) to produce sufficient quantities of the molecule by expression in appropriate host cells or systems and isolating the binding molecules from the host cell or system or from the growth medium or supernatant thereof, as appropriate. Alternatively, said binding molecules or target entities may be produced by other appropriate methods, e.g. by chemical synthesis of the nucleic acid encoding the binding molecule and expression in a suitable host or in an *in vitro* transcription system.

Variants or derivatives of a binding partner or target entity include peptoid equivalents, molecules with a non-peptidic synthetic backbone and polypeptides related to or derived from the original identified polypeptide wherein the amino acid sequence has been modified by single or multiple amino acid substitutions, additions and/or deletions which may alternatively or additionally include the substitution with or addition of amino acids which have been chemically modified, e.g. by deglycosylation or glycosylation. Conveniently, such derivatives or variants may have at least 60, 70, 80, 90, 95 or 99% sequence identity to the original polypeptide from which they are derived.

Variants or derivatives of an antibody molecule further include the conversion of one format of antibody molecule into another format (e.g. conversion from Fab to scFv or vice versa, or the conversion between any format of antibody molecules described elsewhere herein), or the conversion of an antibody molecule to a particular class of antibody molecule (e.g. the conversion of an antibody molecule to IgG or a subclass thereof, e.g. IgG1 or IgG3, which are particularly suitable for therapeutic antibodies).

Variants or derivatives further include the association of binding partner molecules or target entities with further functional components which may for example be useful in the downstream applications of said binding partners or target entities. For example the binding partners or target entities may be associated with components which target them to a particular site in the body, or detectable moieties useful for example in imaging or other diagnostic applications.

Clearly, the main requirement for such components, fragments, variants, or derivative binding partner molecules or target entities is that they retain their original functional activity in terms of binding ability or have improved functional activity.

The binding partner molecules (preferably antibody molecules) or target entities isolated, detected, selected or identified using the methods of the present invention may be used in any methods where binding partners specific to a target entity (for example antibodies specific to a particular antigen) are required. Thus, the binding partners (preferably antibody molecules) or target entities can be used as molecular tools. Also disclosed is a reagent which comprises such binding partner molecules or target entity molecules as defined herein. In addition, such molecules can be used for *in vivo* therapeutic or prophylactic applications, *in vivo* or *in vitro* diagnostic applications, or *in vitro* assays.

The binding partners may be used in therapeutic applications, either in the form isolated from the expression libraries or engineered or converted forms, e.g. it may be desirable to convert an scFv molecule to an IgG or to multimerized peptides. The therapeutic effect might be effected by inducing a biological activity upon the therapeutic molecule showing agonistic or antagonistic binding to the target entity/ligand, e.g. inducing apoptosis (for example of cancer or viral infected cells), inhibiting growth or stimulating detachment from matrix by blocking the natural ligand from binding (to thereby inhibit growth and/or spread of tumours). Such therapeutic effects might be achieved due to properties of the target entity/ligand itself, or a multimer thereof (some receptors are activated by cross linking them).

Where the binding partners selected or identified, etc., are antibody polypeptides then these can be used for *in vivo* therapeutic and prophylactic applications, e.g. to confer passive immunity to particularly susceptible individuals (e.g. immunocompromised patients, small children, the fetus of pregnant women, people in endemic areas for disease, etc). For example if the antibodies are capable of neutralizing infective or disease related agents then these can be administered to an appropriate subject to combat disease. Alternatively, antibodies (or other forms of binding partner) can be attached to other therapeutically effective molecules, e.g. to cytotoxic agents (small molecule or protein), pre-toxin or other drugs and targeted to disease tissue or specific cell types, e.g. tumour cells or virus infected cells. Further therapeutic effects might be achieved by other functions engineered into the agent to be administered like the ability to activate macrophages, complement or cytotoxic T cells conferred for example after changing an scFv to an Ig, generation of bispecific molecules, e.g. linking tumour cells and killer cells.

Alternatively such antibodies (or indeed other types of polypeptide which interact with target entities associated with particular tissue or body sites, or cell types, e.g. tumour cells or virus infected cells) can be conjugated to labels, e.g. dyes, fluorescent or radioactive labels or enzymatically detectable labels, and used for *in vitro* or *in vivo* diagnosis, for example by imaging or standard immunohistochemical procedures. Other preferred uses include theranostic uses (i.e. antibodies or binding partners used in both diagnosis and therapy). In addition, such antibody molecules or other binding partners may be used in affinity chromatography procedures to isolate target entities.

In particular, antibodies to cell surface expressed proteins provide a well described starting point for the successful development of new diagnostic and therapeutic drugs. This is particularly the case in the cancer field, where the knowledge of specific cell surface markers as well as antibodies binding to them, is a clear bottleneck in drug development, and the methods of the invention can be used to identify or select both novel cell surface markers (target entities) and antibodies (binding partners). Currently, only a very limited number of cell surface expressed tumor-associated or tumor-specific epitopes are known. Thus, every new epitope of such type - and of course specific antibodies binding to them - would open new possibilities in the treatment of cancer. Possible applications span from naked whole IgG antibody products for ADCC-based therapy, over recombinant oligo-specific/oligo-valent constructs, to fusion-protein immunotoxins and radiolabeled antibody fragments for tissue-specific and targeted therapy, as well as dyeor radio-coupled antibodies for *in vitro* and *in vivo* diagnosis.

The target entities, or fragments thereof could be used as vaccines, in particular vaccines for use in the treatment for cancers and infectious diseases (depending of course on the target entity in question). The target entity may be used as target for agonists and antagonists, in form of peptides, proteins or small molecule drugs, which may for example block or induce functions like apoptosis or regulating cell growth. The target entities may also be used as a target for further screening, to identify even more molecules able to do some of the things described above. In some cases, the target entities or fragments thereof might also have an effect in itself, like competitively binding of molecules naturally binding to it.

Suitable and appropriate adaptations of the antibody molecules, if necessary for such uses, e.g. the conversion to IgG1 or IgG3 classes for therapy, the incorporation or addition of an appropriate label for imaging, etc., would be well known to a person skilled in the art.

The most suitable antibodies for the various uses described above can be readily identified using appropriate tests which can be designed by a person skilled in the art. For example, in applications where high affinity or avidity of an antibody or a binding partner is important these criteria can readily be tested in candidate antibodies using standard assay techniques (e.g. Biacore assays). In addition, where antibodies against a particular infectious agent, e.g. a bacteria, virus etc., have been identified, appropriate tests can be carried out to assess which antibodies are most effective to neutralise the infectious agent. For example, in the case of bacteria, bactericidal and opsonophagocytic activity against the bacteria in question can be assessed. Similarly, in the case of viruses, viral neutralisation studies can be carried out to identify the best candidates.

A preferred embodiment of the methods of the invention disclosed herein thus concerns the use of an antibody phage display expression library as described herein to isolate, detect, identify or select one or more antibody molecules which bind specifically to one or more target antigens, for example one or more target antigens which are associated with particular diseases, cells, tissues or foreign agents. This could for example be carried out by screening the antibody expression libraries with the target antigens.

Also disclosed are such isolated, detected, identified, selected or manufactured antibody molecules (or other binding partners), or target entities, for use in therapy or *in vivo* diagnosis or for use in any of the other applications mentioned above. Also disclosed is the use of such antibody molecules (or other binding partners), or target entities, in the manufacture of a medicament or composition for use in therapy (in particular therapy for cancer or infectious diseases) or *in vivo* diagnosis or for use in any of the other applications mentioned above. Methods of treatment of a patient comprising the administration of an appropriate dose of such an antibody molecule (or other binding partner), or target entity, are also disclosed.

When said antibody molecules (or other binding partners), or target entities, are used in the above described uses and methods then these may be administered in any appropriate way. For example such antibody molecules (or other binding partners), or target entities, may be administered locally at the site where action is required or may be attached or otherwise associated with entities which will facilitate the targeting of the antibody molecules (or other binding partners), or target entities, to an appropriate location in the body.

Pharmaceutical compositions comprising the antibody molecules (or other binding partners), or target entities, as defined herein, together with one or more pharmaceutically acceptable carriers or excipients are also disclosed.

Also disclosed are methods of diagnosis or imaging of a patient comprising the administration of an appropriate amount of an antibody molecule (or other binding partner), or target entity, as defined herein to the patient and detecting the presence, location and/or amount of the antibody molecule in the patient.

The antibody molecules (or other binding partners), or target entities, identified, selected, etc., from the expression libraries of the invention may equally be used in methods of diagnosis which are carried out *in vitro,* if appropriate, e.g. carried out on a tissue sample or some other kind of sample, e.g. blood, obtained or derived from a patient.

Preferred diseases to be treated or diagnosed, etc., are cancer, infectious diseases caused by infectious agents, inflammatory diseases, autoimmune diseases or degenerative diseases.

The terms "therapy" or "treatment" as used herein include prophylactic therapy. The terms "therapy" and "treatment" include combating or cure of disease or infections but also include the controlling or alleviation of disease or infection or the symptoms associated therewith.

### AffiSelect Method

The AffiSelect method provides a novel technique to indirectly identify the library member (candidate binding partner) via detecting its ligand (or at least by detecting an entity with which the ligand is associated) as opposed to detecting the library member (candidate binding partner). This is of particular value in screening small libraries against complex ligands like live cells, although the applicability of the method is not limited to those applications.

Thus, at its most general the AffiSelect method provides a method of screening a library of molecules (in this case the candidate binding partners which are to be subjected to further analysis) to identify and/or select one or more members thereof which are candidate binding partners for one or more ligands (target entities) comprising:
a) contacting an expression library of candidate binding partners in solution with one or more ligands;
b) capturing ligands which have become bound to one or more members of the expression library onto a solid phase; and
c) detecting the presence of a ligand, thereby detecting the presence of one or more members of the expression library which are candidate binding partners for the ligand.

Thus, when used in combination with the screening methods of the present invention, in order to further analyse the candidate binding partners which have been identified, the AffiSelect method comprises the steps of:
i) contacting one or more of said candidate binding partners in solution with one or more target entities;
ii) capturing target entities which have become bound to one or more of the candidate binding partners onto a solid phase; and
iii) detecting the presence of a target entity, thereby detecting the presence of one or more candidate binding partners for the target entity.
Thus, taking the terminology used in the description of the screening methods of the present invention, in the discussion of the AffiSelect method below, any reference to an expression library can be taken to refer to one or more of the candidate binding partners which are to be subjected to further analysis and any reference to ligands can be taken to refer to target entities.

One of the main requirements for the expression libraries (in this case the candidate binding partners which are to be subjected to further analysis) to be used in the AffiSelect method is that they must be in solution when they are initially brought into contact with the ligands (target entities) against which they are being screened. By "in solution" is meant that the expression libraries are not attached to a solid phase when they are initially brought into contact with the ligands against which they are being screened, i.e. the initial expression libraries are non-immobilized expression libraries.

The expression library (candidate binding partner) constructs for use in the AffiSelect method (and therefore also the candidate binding partner constructs making up the expression library screened in step (a) of the methods of the present invention) may optionally additionally contain other appropriate components, for example origins of replication, inducible or non-inducible promoters for initiating transcription, enhancers, antibiotic resistance genes and markers, general tags or reporter molecules, primer binding sites to enable amplification of the constructs by e.g. PCR, or other desirable sequence elements. Appropriate sources and positioning of such additional components within the library constructs so that they perform their desired function would be well within the normal practice of a skilled person in the art.

The inclusion of general tags or markers in the library constructs and in the expressed library members (candidate binding partners) is particularly important in the expression libraries for use in the AffiSelect method and such tags are used to facilitate the binding of library members (candidate binding partners) to a solid phase in the capture step of the method, i.e. are used to facilitate the capture step. Any appropriate tag may be used in this regard providing it can facilitate binding of the library members (candidate binding partners) to a solid phase. However, conveniently such tags are affinity molecules which can facilitate binding to a solid phase by binding to a partner affinity molecule immobilized directly or indirectly onto the solid phase. Exemplary tags may be c-myc tags (which can for example be captured via an anti c-myc antibody) or His tags (which can for example be captured via a Nickel surface) or biotin (which can for example be captured via streptavidin molecules), or a phage surface protein such as gp8, (which can for example be captured via an anti gp8 antibody), or antigen peptide tags such as FLAG or HA which may be recognized by an antibody. Such tags or markers may conveniently also be directly or indirectly detectable. Such tags or markers are typically general tags or markers which are present in all the library constructs and can be used to capture the library members onto a solid phase. Such tags or markers are not required for the detection step of the AffiSelect methods (as detection is carried out via the detection of the ligand rather than the library member, see in more detail below). However, if desired such tags can be used to detect the presence of library members. Their presence can prove extremely useful in determining the affinity of a ligand for a library member.

Once the appropriate expression library (candidate binding partner) constructs have been obtained at the nucleic acid level, these can then be expressed for screening and for use in step (i) of the method in appropriate expression systems. Appropriate conditions and methods of expression are well known and described in the art.

The target entities for use in the AffiSelect method can take any of the forms as described above. Thus, for example, the target entities may be cell surface molecules provided for example as a component of whole cells or as membrane fractions of cells, or may be an isolated target entity of interest attached to appropriate solid phase. Preferably said target entities are cell surface molecules. Conveniently, as the AffiSelect method is used for further analysis of initial candidate binding partners identified by the organic phase separation screening methods described herein, the target entities can be the same as those used in step (a) of these methods.

Whether the ligand (target entity) is a cell surface molecule or a molecule of interest attached to an appropriate solid phase, for use in the AffiSelect method the ligands contain or are associated with a reporter moiety to enable detection of the ligands. Such reporter moieties are preferably nucleic acid molecules, more preferably DNA molecules, which can then be detected by nucleic acid based methods. A preferred method of detection is by PCR but any appropriate method can be used, for example hybridisation of a labelled probe. Alternatively, the ligand may be attached to a non-magnetic fluorescent bead, or to another population of beads of a distinguishable size that can be counted/detected in microscopy or by a Coulter Counter ZM (Coulter Electronics Ltd.). Thus, it can be seen that such reporter moieties can take a large variety of different forms providing detection of said reporter moieties is possible.

Such reporter moieties may be inherently associated with the ligands (target entities). For example, if the ligand (target entity) is a cell surface molecule, then a preferred reporter moiety to be detected would be a DNA molecule, e.g. a gene (i.e. a reporter gene) which is present in the cells on which the ligand is expressed/displayed. Appropriate and preferred reporter moieties are therefore moieties which are present in all cell types, e.g. a housekeeping gene such as β-action.

Alternatively an exogenous reporter moiety may be associated with the ligand (target entity). For example, in the case of cells, an exogenous reporter moiety (e.g. a reporter gene) may be introduced into the cells by standard methods, e.g. transfection, including but not limited to lipofection, retroviral systems or electroporation.

If the ligand (target entity) is a naked molecule, e.g. a naked polypeptide then this may be tagged or associated with an appropriate reporter moiety using methods well known and described in the art, for example if the ligand is associated with its encoding nucleic acid or another nucleic acid based tag, e.g. as described above, the reporter moiety can conveniently be provided within this nucleic acid molecule. If the ligand is a molecule, e.g. a polypeptide, attached to a particulate solid phase then said reporter moiety might be attached to the solid phase separately to the polypeptide, for example using similar methods to those described above. In particular, if streptavidin beads are used then the reporter moiety is preferably a biotinylated DNA fragment comprising a site or region which can facilitate detection of the reporter moiety, e.g. a primer site to enable PCR amplification of a fragment of the reporter gene or a probe binding site. Each bead can then carry a mixture of biotinylated reporter moiety and biotinylated polypeptide (or other ligand).

It can be seen therefore that such "association" between a reporter moiety and the ligand (target entity) may comprise a direct interaction between the reporter moiety and the ligand, i.e. the reporter moiety and the ligand are part of the same molecule, e.g. the ligand protein and the reporter moiety are conjugated to one another as part of the same molecular entity. Alternatively, such "association" may be indirect, e.g. the reporter moiety is attached to or contained within a moiety to which the ligand is attached, e.g. is contained within the cell on which the ligand is expressed or displayed or immobilized onto the solid phase to which the ligand is attached.

Such reporter moieties are preferably general reporter moieties which are associated with all the ligands (target entities) being displayed and can thus be used as a general reagent to detect the presence of any and all target ligands (and thereby the presence of a candidate binding partner).

The step of bringing the ligands (target entities) into contact with the expression library (candidate binding partners) or vice versa in the AffiSelect method can be carried out in any appropriate way under conditions such that appropriate candidate binding partners can interact with or bind to the ligands (target entities) which are present. Such conditions will generally vary depending on the nature of the expression library (candidate binding partners), the ligand (target entity) and the moiety with which the ligand (target entity) is associated. However, appropriate conditions to facilitate binding can be readily determined by a person skilled in the art. Such a "contacting" step will generally occur in an appropriate solution or aqueous medium. Thus, if the ligand is present on a cell which is cultured as a monolayer or cells which are obtained from a mammalian subject, e.g. from a mammalian organ or tissue, then these are generally harvested and resuspended in an aqueous medium by appropriate techniques before being contacted by the expression library.

Importantly, it has been found that in the Aflselect method the contacting step can be carried out in bacterial expression medium, for example L Broth, as well as in standard aqueous media such as PBS. This is important and surprising because it means that library members (candidate binding partners) can be expressed in bacteria (e.g. by culturing them in a bacterial expression medium such as L Broth) and clones can be taken directly from the expression plate for use in the AffiSelect screening methods. Preferably such bacterial expression medium is made isotonic (e.g. to approximately 140 mM NaCl, (LB is usually approximately 171 mM NaCl)) before the ligands (target entities) are added but after expression of the library members has been induced. This step of making the bacterial medium isotonic is especially preferred when the ligands (target entities) are cell surface molecules on live cells rather than on beads.

Exemplary "contacting" conditions may comprise incubation on ice or at 4°C for between 30 minutes and 4 hours. However, these may be varied as appropriate depending on the nature of the ligands, expression libraries, etc. The expression library - ligand mixture may optionally and preferably be subjected to gentle rocking, mixing or rotation. In addition other appropriate reagents such as blocking agents to reduce non specific binding may be added. For example 1-4% BSA or other suitable blocking agent (e.g. milk) may be used. It will be appreciated however that the contacting conditions can be varied and adapted by a skilled person depending on the aim of the screening method. For example, if the incubation temperature is increased, for example to room temperature, this may increase the possibility of identifying binders to a different subset of ligands, e.g. binders to cell surface proteins which are readily internalized. Again such adaptations to the conditions are within the ambit of the skilled person.

Conveniently the number of copies of ligands (target entities) can be varied by altering the number of moieties present with which the ligands are associated. For example, when the ligand is a cell surface molecule or a molecule attached to a solid support, then the number of target ligands can be adjusted by increasing or decreasing the number of cells or solid supports present in the contacting mixture. The number of ligands required can readily be determined by trial and error, but for example when the ligand is a cell surface molecule 5 000 to 200 000 cells are conveniently used. Indeed it is an advantage of the AffiSelect method that it is sensitive enough to work with cell numbers as low as 5 000 or 1000 cells (or even lower, e.g. the use of PCR as a method of detection should potentially enable the detection of a single cell). In this regard, particularly when screening with live cells derived from mammalian subjects, e.g. human patients, such cells are often rare resources and may not be available in large numbers. Thus, to be able to carry out screening on such low numbers of cells is an important advantage. It has been shown that where detection takes place by PCR and the candidate binding partners are antibodies, only 40 pg of antibody is required to enable detection. This means that the AffiSelect method should be suited for analysing candidate binding partners for ligand molecules which are expressed at a very low level, for example when less than 1000 molecules are expressed.

One or more types of candidate binding partners (i.e. one or multiple candidate binding partners) may be present in the same reaction vessel or assay compartment (e.g. assay well) for contacting step (i) of the AffiSelect method (and hence in the following steps of the method). Preferably however only one type of candidate binding partner is present in the reaction vessel or assay compartment for the contacting step (i).

Once ligands (target entities) have been contacted with the expression library (candidate binding partners) under conditions such that the ligands can become bound to one or more members of the expression library, the ligands which have become bound in this way are then captured onto a solid phase to facilitate their isolation or removal or purification from the other components of the reaction mixture such as nonbound ligands. In other words, during the capture step only the ligands (target entities) which are bound to an expression library member (candidate binding partner) are captured onto the solid phase.

Preferred solid phases in this regard are particulate solid phases which facilitate easy manipulation and washing. Magnetic solid phases and in particular magnetic particles/beads are especially preferred. In order to distinguish these solid phases from the solid phases to which the ligands (target entities) may be attached (as described above), such solid phases can be referred to as "capture solid phases". Suitable magnetic beads are available commercially from Dyno Specialty Polymers AS of Lillestrøm, Norway and Dynal Biotech ASA. Particular examples of magnetic beads which can be used in the methods described herein are the M-450, M-270 or M-280 beads from Dynal Biotech ASA, Norway.

Ligand (target entity)-library member (candidate binding partner) complexes can be captured onto a solid phase by any appropriate method. A preferred method in accordance with the AffiSelect method involves the interaction of a capture molecule on the solid phase with a partner molecule or tag associated with the members of the expression library (candidate binding partners) i.e. the capture step is facilitated by a molecule or tag on the candidate binding partner. In this way only ligands which are bound to members of the expression library are captured onto the solid phase. All ligands not binding to members of the expression library can, if desired, be removed by one or more steps of washing the solid phase, or simply by separating the solid phase from the reaction mixture after capture has taken place. Thus, it can be seen that as the capture step is facilitated by the expression library members rather than the ligands, the isolation of the solid phase from the rest of the mixture will allow the removal of ligands which have not bound to library members.

The interaction between a capture molecule on the solid phase and the partner molecule or tag on the expression library member is conveniently based on an affinity interaction, although any other appropriate reaction may be used. For example, the members of the expression library can be engineered to express one component of the affinity interaction and the other component can be attached to the solid phase by any appropriate means. Preferred examples of such affinity interactions are antibody-antigen interactions, streptavidin-biotin interactions.

Streptavidin coated beads are commercially available and these can be used to capture ligand-library member conjugates via the presence of a biotin molecule in the library member part of the conjugate. Alternatively, if the expression library is a phage library then capture can readily be achieved using a solid phase to which an antibody to a phage surface protein has been attached, e.g. anti gp8. Alternatively the library members may be engineered to express a tag which is recognized by an affinity partner that facilitates binding to the solid phase. Examples of appropriate tags are c-myc (or other antigen peptide tags) or His tags which can be recognized by anti c-myc antibodies (or other appropriate antibodies) and metal ions (for example Ni²⁺), respectively, which can in turn facilitate capture to the solid phase. Where antibodies are used to facilitate capture, these can be attached to the solid phase either directly or indirectly, e.g. via an appropriate secondary or tertiary antibody such as an anti IgG antibody. Capture can be effected in any appropriate way depending on the reagents used. Thus, the antibodies may be attached to the solid phase, which is then brought into contact with the mixture containing ligand (target entity)-library member (candidate binding partner) complexes. Alternatively, the antibodies may be allowed to bind to the library members in the reaction mixture before the antibodies are bound to the solid phase (e.g. via a secondary antibody) to facilitate ligand-library member capture. Indeed, this is a preferred embodiment of the AffiSelect methods. Appropriate capture conditions in terms of temperature and time can readily be determined by a person skilled in the art. However, exemplary conditions may comprise incubation for 10 minutes to 2 hours at room temperature or 4°C (depending on the stability of the ligand or library member), preferably with gentle rocking, mixing or rotation. The amount of solid phase to include, or the ratio of solid phase to ligand to include in order to facilitate capture of the ligand - library member complexes can be readily determined by a person skilled in the art.

One or more washing steps can be carried out at any appropriate stage in the AffiSelect method. For example, as described above, a washing step (or at least a separation step) is carried out after step (ii) in order to remove ligands (target entities) which have not bound to expression library members (candidate binding partners). One or more steps of washing the moieties with which the ligands are associated (e.g. washing the cells or the solid phases with which the ligands are associated), might also be carried out after step (i), in order to remove expression library members which have not become bound to ligand. Indeed, such washing steps are preferred. Also, one or more washing steps may be carried out on the moieties with which the ligands are associated, or on the solid phases, at other appropriate times during the course of the method, e.g. to remove non bound entities. How many wash steps to include can readily be determined by a person skilled in the art.

The step of detecting the presence of a ligand (target entity) is carried out by detecting the ligand directly or at least by detecting an entity (not the library member) with which the ligand is associated. This step provides a contrast to prior art methods where detection generally occurs by detection of the library member which is bound to the ligand rather than the detection of ligand.

Detection of the presence of a ligand/target entity (and therefore the presence of a ligand-library member complex, as the non-complexed ligands should have been removed by the capture step as they will not have become bound to the solid phase) is conveniently carried out by detecting the presence of a reporter moiety on or associated with the ligand. Appropriate reporter moieties are discussed above. As described above, said reporter moieties are usually present on or associated with all ligands, i.e. are general labels for all ligands. Thus, as the same reporter moiety is present on (or associated with) each ligand, the presence of a positive signal indicates the presence of a ligand-library member conjugate and therefore a library member which is a candidate binding partner for a ligand. It should be noted however that if the reporter moiety is present within a cell, i.e. in embodiments where the ligand is a cell surface molecule, then this will have to be exposed, e.g. by cell lysis or other means of cell disruption, before detection can take place. Thus, in these embodiments of the invention, where the AffiSelect methods are used for further analysis of candidate binding partners, the presence of a positive signal provides a further indication of the presence of a candidate binding partner which potentially binds the relevant target entity. If more than one candidate binding partner is present in the detection reaction then further analysis can be carried out to determine which of the candidate binding partners bind to the target entity. Preferably however, as discussed above, only one type of candidate binding partner will be present in the detection reaction and thus the presence of a positive signal will indicate the presence of a candidate binding partner which potentially binds the relevant target entity.

Appropriate methods of lysing/disrupting cells to expose the reporter moieties which are for example present in the nucleic acids contained within the cells, are well known and described in the art. A preferred lysis buffer for use in the AffiSelect method involving eukaryotic cells contains proteinase K, which digests protein and can remove histones from the DNA before the detection step. Other proteases might also be included in the lysis buffers for use in the AffiSelect method.

As described above PCR is the preferred method of detection in the AffiSelect method. However other appropriate methods of reporter moiety detection could be used, e.g. probe hybridisation, fluorescent beads, different size beads, etc (see above). PCR is preferred because of the high sensitivity of the method (even one ligand (or cell) may be detected using PCR), and also because it allows information to be obtained about the ligand, for example sequence information (if for example the ligand is associated with the nucleic acid encoding it), or identification of the ligand if for example the ligands are tagged with DNA molecules to enable identification of the ligand, e.g. tagged with DNA molecules of different sizes, or tagged with DNA molecules with specific sequence tags which can be used to specifically identify the ligand:

Conveniently the PCR reaction is carried out in solution (although it could also be carried out on a solid phase) and the presence or absence of a PCR product detected in standard ways, such as on an agarose gel (for example the E-gel 96 system, Invitrogen, makes it possible to analyse 96 samples in 20-30 minutes). Generally the samples are well mixed before carrying out the PCR (or other detection step).

Appropriate PCR primers to amplify all or a portion of the reporter moiety are selected according to the sequence of the reporter moiety by methods well known and described in the art. If the nucleic acid content of the assay wells is appropriate, for example if the ligand is encoded for by nucleic acid which has been transfected into cells using an appropriate expression vector (which contains an appropriate reporter moiety), or the ligand is associated with the DNA encoding it, then PCR primers may be selected to amplify the nucleic acid encoding the ligand sequence as well as the reporter moiety. This can be advantageous in that the PCR products can be cloned directly into an appropriate vector for sequencing, thereby resulting in the identification of the ligand sequence.

The detection of a positive signal, by PCR or otherwise, is indicative of solid phases which require further investigation. For example, if PCR is carried out in solution in the wells of an assay plate, a positive signal is indicative that the well or wells in question contain at least one potential binding partner for a ligand (target entity) which can then be subjected to further analysis and investigation. For example, the bacterial colonies (or other library members) present in that well can be subjected to thorough screening, e.g. by cell-ELISA to identify the colony (library member) which expressed the candidate binding partner for the ligand. Appropriate analysis can then be carried out to further identify and characterise the binding partner at the gene and protein level.

A positive signal, generated by PCR or otherwise, is conveniently determined by comparison to a background signal or a known negative signal. Means and methods of measuring and comparing said positive, background, and/or negative signals are well known to a skilled person. In embodiments where the target entities are cell surface modules, preferred negative signals for comparison are provided by carrying out the screening on irrelevant or negative cells, e.g. cells which do not express the target entities on their surface, or express the target entities only at low levels.

The invention will now be described in more detail in the following non-limited Examples with reference to the following Figures in which:
Figure 1 shows FACS analysis of candidate antibody clones 1 (scFv1) and 2 (scFv2) selected by the methods of the present invention on mammary tumour cells and demonstrates that both these clones bind to mammary tumour cells thereby demonstrating that target antigens for both these clones are expressed on mammary tumour cells.
Figure 2 shows a table of FACS analysis results to assess the binding of candidate antibody clones 1 (column 2) and 2 (column 1) to different cell lines. The results show that clone 1 is specific to the breast carcinoma cell lines tested whereas clone 2 binds to tumour cell lines of different origin.
Figure 3 shows polyclonal phage ELISA on lung carcinoma cell line A-549 with phage dilutions after panning round 1-4 (R1-R4) using 2 washes and organic phase centrifugation (org) and lectin coated magnetic beads (beads).
Figure 4 shows an example of AffiSelect PCR results showing amplified beta-actin DNA in cases where the tested scFv expression was binding to cells (A-549, HUVEC and PBL). Results of the same scFv expression samples are shown (in the same order) on the three cell types. Arrows mark the scFv expression samples that showed tumor-specific DNA amplification.
Figure 5 shows a FACS result of one of the scFv found after panning using 2 washes and organic phase centrifugation. The scFv was tested on the same cell types that were used in the panning and pre-panning steps (A-549, HUVEC and PBL).
Figure 6 shows a filterscreening result for the antigen identification with scFv 2. Five tumor cDNA expression samples are shown (circles) with high binding signals towards the scFv 2-coated filter. These samples were negative on a PBS/3% BSA-coated filter (not shown).

### EXAMPLES

### Materials and methods

### Cells

Two human breast carcinoma cell lines (PM-1 and MT-1, kindly provided by the Department of Tumorbiology, Norwegian Radium Hospital (DNR), Oslo, Norway), lung carcinoma cell lines (A-549, ATCC CCL-185, kindly provided by Viventia Biotech Inc.; SW900, ATCC HTB-59, kindly provided by the Department of Tumorbiology, DNR), and an endothelial cell line (HUVEC, ATCC CRL-1730, kindly provided by Viventia Biotech Inc.) were used for panning experiments. Both breast carcinoma cell lines were cultured in RPMI-1640 medium (Cambrex) supplemented with 10% FCS (Gibco BRL), 2mM L-glutamine (Cambrex) and 10mM HEPES (Cambrex). A-549 was cultured in Ham's F12 (BioWhittaker) supplemented with 10% FCS and 2mM glutamine. The endothelial cell line was cultured in RPMI-1640 medium supplemented with 10% FCS, 15 mg/l endothelial growth factor supplement (Sigma) and 50 mg/l heparin (Sigma). Cells were cultured at 37°C with 5% CO2 and a humidified atmosphere. For each experiment cells were washed with PBS, harvested with 1mM EDTA in PBS, washed once with PBS and counted. For panning experiments, cells were either resuspended in PBS/3% BSA (Sigma) or PBS/4% skimmed milk (Merck).

Peripheral blood lymphocytes (PBL) were isolated from fresh human blood or buffy coats using lymphoprep (Axis-Shield; see manufacturer's protocol for details). Cells were washed once with PBS, counted and resuspended in PBS/3% BSA or PBS/4% skim milk.

### Lectin-coated magnetic beads

M-450 epoxy beads (Dynal) were coupled with lectin (WGA: Wheat Germ, *Triticum vulgaris;* Calbiochem) according to manufacturer's protocol. In short, epoxy-beads were washed and subsequently incubated in 0.1M borate buffer, pH 8.5 with 10µg lectin/10⁷ beads rotating overnight at room temperature. Beads were washed three times with PBS/0.1% BSA and stored at 4°C in PBS/0.1% BSA/0.02% sodium azide at 4x10⁸ beads/ml.

### Phage scFv display library

For the panning experiments an IgM scFv library was used that was constructed from PBL of 6 healthy donors and cloned into a phagemid display system based on pSEX 81 (Welschof et al., 1997, PNAS, 94:1902-1907, Løset, et al., 2005, J. Immunol. Methods 299:47-62) after N-terminally moving the HindIII site of the linker, resulting in a 21 amino acid linker sequence.

### Panning/Contacting Step

To remove phages binding to common cell proteins 3.75x10¹² phages (150µl library of approx. 2.5x10¹³ cfu/ml) was incubated with 2x10⁵-2x10⁶/ml cells of non-tumor origin in a blocked tube rotating for 1 hr at 4°C (= negative pre-panning). As blocking reagent either PBS/3% BSA or PBS/4% milk were used.

In one project PBL were used for the negative pre-panning step. After negative pre-panning the PBL were washed twice with block solution, after which the reduced phage library plus two wash-supernatants were incubated with breast tumor cells as described above (panning).

In another project two negative pre-panning steps were performed one after the other. In a first pre-panning step, the library was incubated with PBL as described above. After the first pre-panning the PBL were washed twice with block solution, after which the reduced phage library plus two wash-supernatants were incubated with HUVEC in a blocked tube rotating for 1-2 hr at 4°C as a second negative pre-panning step. After two washes of the second negative pre-panning, the supernatant plus wash-supernatants were incubated with lung tumor cells as described above (panning).

### Washing/Separation

Four methods were tested to separate free phages from cell-bound phages:
1) Single step organic phase separation according to the BRASIL method (Giordano et al., 2001, *supra*):
   After panning, 2x150µl of the tumour cell suspension were directly layered on top of 2x300µl organic phase solution (dibutyl phthalate:cyclohexane 9:1 [v:v]; d=1.03 g ml⁻¹). After centrifuging at 10.000g for 10 min. at room temperature (RT), the tubes were snap frozen in liquid nitrogen, the bottom of the tube was sliced off and the cell pellet was transferred to another (blocked) tube.
2) As method 1, but with one or two washing steps with 1 ml PBS/0.4% BSA before resuspension in 300µl PBS/0.4% BSA and organic phase centrifugation.
3) Classical method:
   After panning, the tumor cells were spun down (500g, 5 min. 4°C) and the pellet was washed five times with 1 ml PBS/0.4% BSA.
4) Using magnetic beads
   After panning, the tumor cells were spun down (500g, 5 min. 4°C), resuspended in PBS/0.1% BSA at 5x10⁶cells/ml and incubated with lectin-coated beads (1:4 cell:bead ratio) rotating for 20 minutes at 4°C. The beads were washed 10 times in 1 ml PBS/0.1% BSA using a magnet and resuspended in 200µl PBS/0.1% BSA.

### Infection

Cell pellets or beads were added to 10 ml E.Coli XL1 blue in log phase and incubated at 37°C for 15 min. at 60 rpm, then for 45 min at 200 rpm. Bacteria were plated at different concentrations on LB_{TAG} plates (LB added 30µg/ml tetracyclin (T), 100µg/ml ampicillin (A) and 100mM glucose (G)) for evaluation and on two large rectangular plates (243x243x18 mm; Nunc) for later packaging. All plates were incubated overnight at 37°C.

### Packaging

Bacteria from the two large plates were harvested by scraping. The bacteria were propagated in liquid culture, and the phagemid in those cells was packaged into phage particles with M13 helper phage (Stratagene), overnight at 30°C. The next day, phages were precipitated with PEG/NaCl and resuspended in 1 ml PBS of which 100µl phages (approx. 10¹³ cfu) were used for a new round of panning.

### Polyclonal phage ELISA

After blocking 30 min. 4°C with PBS/3% BSA, tumor cells and PBL were incubated for 1 hr at 4°C with 100µl phages (1:10-1:10⁶ dilutions) from each panning round. After 3 washes with PBS, the cells were incubated for 1 hr at 4°C with 100µl rabbit anti-Fd (Sigma; 1:4000) antibody. After washing 3 times with PBS, the ELISA was developed with a monoclonal horseradish peroxidase (HRP) conjugated goat-anti-rabbit Ig antibody (Dako; 1:4000) and the substrate ABTS (Calbiochem). The absorption was measured at 405 nm. (Similar methodology was used where appropriate to carry out ELISA assays with single phage as opposed to polyclonal phage).

### Expression and purification of scFv in E.Coli

ScFv DNA from the panning round that showed an increase in tumor-specific binding (polyclonal phage ELISA) was cloned into the secretion vector pHOG21 (Kipriyanov et al., 1997, J. Immunol. Methods, 200: 67-77) and individual scFv clones were expressed in E.Coli XL-1-Blue as described previously (Dörsam et al., 1997, FEBS Letters, 414: 7-13) or in 96 deep well format (100-200 microliter volume). Deep well expressions were performed overnight at 30°C with 1mM IPTG (Sigma). The next day the plates were centrifuged for 10 min. at 4000rpm, 4°C and the supernatants (containing the expressed scFv) were used in further experiments. In case the expression samples were to be used in AffiSelect, the polyclonal collection of cloned scFv DNA was transformed into competent XL-1 Blue E.Coli and plated on LB-TAG 22x22 cm bioassay trays (Coming). Individual clones were picked using QPix2 robot (Genetix, 1td., UK) into 384-well plates containing LB-TAG growth medium [30µg/ml tetracyclin (T), 100µg/ml ampicillin (A), 1% (w/v) glucose (G)] added - 4% glycerol and grown overnight at 37°C. Single scFv clones were re-inoculated in 384-well plates containing LB-TAG medium and expressions were done with 100µM IPTG. The next day 50µl PBS/3% BSA was added to each well (containing 100µl expression) before centrifuging the plates.

### Expressed scFv were tested for their binding to cells using 5 different methods:

### 1) ScFv ELISA

After blocking 30 min. 4°C with PBS/3% BSA, tumor cells and PBL were incubated for 1 hr at 4°C with scFv (100µl deep well expression or 10µg/ml purified scFv). After 3 washes with PBS, the cells were incubated for 1 hr at 4°C with 100µl mouse monoclonal HRP conjugated anti-c-myc (Invitrogen; 1:4000) antibody. After washing 3 times with PB S, the ELISA was developed with the substrate ABTS and measuring the absorption at 405 nm.

### 2) 8200 Cellular Detection System (Applied Biosystems),

5µl of 96 deep well scFv expressions were tested in 384 well plates containing 5x10e4 tumor cells (PM-1) or PBL and anti-c-myc-FMAT Blue (AppliedBiosystems) or a combination of mouse anti-c-myc antibody (Invitrogen; 1.25µg/ml) plus goat anti mouse IgG-Alexa Fluor R647 (Molecular Probes; 2.5µg/ml). After 2-4 hour incubation, binding of the scFv to the cells was measured using the 8200 Cellular Detection System, with the signal intensities of only secondary antibody- and irrelevant scFv binding as negative controls.

### 3) AffiSelect

5µl of 96 deep well scFv expressions were tested on tumor cells (A-549), PBL and HUVEC using the Affiselect method. For this, 2.2x10⁴ cells were pelleted (1600 rpm, 4°C, 5 min.) and incubated shaking for 1 hour at 4°C together with 5µl expression sample and 45µl PBS/3% BSA. The cells were washed with 150µl PBS/3% BSA and centrifugation (1600 rpm, 4°C, 5 min.). After the adding 50µl anti-c-myc antibody (Invitrogen; 1:500) to the pellets, the plates were incubated shaking for 1 hour at 4°C.

The cells were washed twice as described before and incubated shaking for 1 hour at 4°C together with 25 µl M-450 pan mouse IgG bead (Dynal) suspension of 5x 10⁶ beads/ml (cell:bead ratio = 1:5). After washing the beads twice with 100µl PBS/3% BSA using a magnet, they were resuspended in 100µl PBS/3% BSA and transferred to a PCR plate.

The bead samples were incubated overnight at 55°C in 30µl PCR buffer containing proteinase K (200µg/ml), 0.45% Tween 20 and 0.45% NP40, and stored afterwards at -20°C until use. After heat-inactivating the proteinase K for 30 min. at 95°C, the samples were placed on a magnet and 5µl of the supernatant was used for PCR with human beta-actin specific primers. In this assay, the presence of a beta-actin PCR product in a well signifies the possible presence of an scFv expression clone which has bound to cells.

### 4) FACS

1-5x10⁵ cells were stained for 1 hour at 4°C with 50µl scFv deep well expression. After washing 3 times with 150µl PBS and centrifugation (1600 rpm, 5 min, 4°C), the cells were incubated with 50µl FITC-conjugated anti-c-myc antibody (Invitrogen; 1:30) for 1 hour at 4°C. Cells were washed 3 times as described above, fixed with 200µl PBS/1-2% formalin (Sigma) and stored at 4°C until being measured with a FACS machine (Coulter).

### 5) Guava

1x10⁵ cells were stained with 50µl scFv deep well expression as described for FACS. To avoid internalization, in some cases cells were fixed with 50µl PBS/1-2% formalin (30 min, 4°C) and washed three times with PBS (1600 rpm, 5 min., 4°C) before incubating the cells with the scFv expressions. Visualization of the bound scFv was performed with an FITC-conjugated anti-c-myc antibody as described for FACS, or with a combination of anti-c-myc antibody (1 hour, 4°C; 5µg/ml; Sigma) followed by three PBS washes as described for FACS and an incubation with a FITC-conjugated anti mouse IgG antibody (1 hour, 4°C; 1:200; Dako). After three PBS washes and fixation as described for FACS, the samples were stored at 4°C until being measured with a Guava EasyCyte (Guava Technologies).

### Cellular filter screening experiments

### Test-strips

To determine the optimal concentration of cells for immobilization onto nitrocellulose membranes (Schleicher & Schuell), test strips (approx. 6x4 cm) were coated for 2 hrs at RT with different amounts of cells (5x10⁴-5x10⁶ cells/ml) in PBS. After washing twice with PBS, the membranes were incubated with a mouse monoclonal anti-MHC class I antibody (Dako; 1:600) for 1 hr at RT. Coated cells were visualized by incubation with a HRP-conjugated rabbit-anti-mouse Ig antibody (Dako; 1:10.000) and ECL detection (Amersham).

### Test array

ScFv clones that have been shown earlier to be positive in cell ELISA were tested in a filter screening assay (Stacy et al., 2003, J. Immunol. Methods, 283:247-259). In short, scFv clones were arrayed onto a nitrocellulose filter. After overnight expression with IPTG the scFv were tested for their binding to a cell-coated filter. The filters were incubated with a mouse monoclonal anti-MHC class I antibody (1:600) and binding was visualized by an HRP- conjugated anti-c-myc antibody (1:10.000) and ECL detection. Five different blocks (PBS/2% BSA, PBS/4% skim milk, SuperBlock (Pierce), Casein Blocking Buffer (Sigma), 2% Blot Block (BDH)) were tested for their reduction in background signal.

### Antigen identification

### Construction of tumor cDNA library and panning,

A cDNA phage display library was constructed as described before (Fosså et al., 2004, Cancer Immunol. Immunother., 53:431-438) from tumor cell line SW900. To find the antigen corresponding to a scFv of interest, biopanning was performed on scFv coated maxisorp tubes (Nunc). In short, maxisorp tubes were coated rotating overnight at 4°C with 10µg/ml scFv in PBS/3% BSA or PBS/4% milk. The next day the cDNA phage display library was blocked for 15 minutes in PBS/3% BSA or PBS/4% milk before adding into the scFv coated tubes. After incubating the library rotating for 2 hr at RT, the tubes were washed 10x with PBS/0.05% tween followed by 10 washes with PBS. Binding phages were eluted by incubating the tubes 5 minutes rotating with 50µl 100mM TEA (Sigma). After neutralizing the eluted phages with 500µl 1M Tris-HCl pH 7.5, 500µl was used for the infection of 10ml XL1-Blue (as described above). After packaging as described previously, the phages were used for a new round of panning on scFv coated tubes. Recovered phages from 3 rounds of panning were tested in a polyclonal phage ELISA (see above) on scFv coated maxisorp plates (Nunc).

### Filter screening of expressed tumor cDNA clones

The tumor cDNA from the panning round that showed an increase in scFv-specific binding (polyclonal phage ELISA) was amplified by PCR and cloned into a modified secretion vector pHOG21 containing an HA tag. Tumor cDNA clones were arrayed onto a nitrocellulose filter and after overnight expression with IPTG tested for their binding to a scFv coated filter (de Wildt, RM, et al., Nat. Biotechnol. 2000, 18(9), 989-994). The filters were incubated with a mouse monoclonal anti-HA antibody (0.2µg/ml; Roche) and binding was visualized by an HRP- conjugated rabbit anti-mouse antibody (1:10000; Dako) and ECL detection.

### Example 1- Testing the BRASIL method

Two breast cancer cell lines and PBL were centrifuged through an organic phase after 1-2 hour incubation with the phage-library, and their recovery in aqueous (upper phase) and organic layer (pellet) after organic phase centrifugation was tested. As a negative control 10¹¹ phages were centrifuged to evaluate the number free phages that slip through the organic phase.

Recovery after panning on human tumor cell lines (PM-1 and MT-1) and PBL was tested. It turned out that PBL have to be freshly isolated. Thawed PBL from a frozen aliquot tend to become sticky during the panning procedure. After centrifugation 75 % (PM-1), 97 % (MT-1) and 71 % (PBL) was found in the cell pellet of the organic phase. These percentages are too high to have a biological significance: it is expected that only a few percent of a whole naive antibody library will bind to cell surface proteins of a given cell type. The number of free phages that slip through the organic phase was only 2x10⁻⁵ % and can be ignored in the future.

Thus, the BRASIL method turned out to be not useful as such: by using only organic phase centrifugation as the separation step, too many (non-specific) binders were found in the cell pellet.

### Example 2 - Comparison of four methods to separate free phages from cellbound phages

1) Single step organic phase separation based on the BRASIL method (Giordano et al., 2001):
   After panning, 2x150µl of the tumour cell suspension were directly layered on top of 2x300 µl organic phase solution (dibutyl phthalate:cyclohexane 9:1 [v:v]; d=1.03 g ml⁻¹). After centrifuging at 10.000g for 10 min. at room temperature (RT), the tubes were snap frozen in liquid nitrogen, the bottom of the tube was sliced off and the cell pellet was transferred to another (blocked) tube.
2) As method 1, but with one or two washing steps with 1 ml PBS/0.4% BSA before resuspension in 300µl PBS/0.4% BSA and organic phase centrifugation.
3) Classical method:
   After panning, the tumor cells were spun down (500g, 5 min. 4°C) and the pellet was washed five times with 1 ml PBS/0.4% BSA.
4) Magnetic beads:
   After panning, the tumor cells were spun down (500g, 5 min. 4°C),
   resuspended in PBS/0.1% BSA and incubated with lectin-coated beads (1:4 cell:bead ratio) rotating for 20 minutes at 4°C. The beads were washed 10 times in 1 ml PBS/0.1% BSA using a magnet and resuspended in 200µl BS/0.1% BSA.

In a first experiment the new method (2) was compared to the BRASIL method (1) and the classical method of 5 washing steps (3). The tumour cells used were the PM-1 breast carcinoma cell line and negative pre-panning with PBL was carried out as described in the Materials and Methods section.

In another experiment a direct comparison was made between method (2) and the use of lectin-coated magnetic beads to separate free phages from cell-bound phages (method 4). The tumour cells used were the A-549 lung carcinoma cell line and negative pre-panning with PBL and HUVEC was carried out as described in the Materials and Methods section.

### Comparison of methods 1, 2 and 3:

After 4 rounds of panning, separation (using method 1, 2 or 3, as appropriate), infection and packaging, a polyclonal phage ELISA was performed separately on phages recovered from each of the 4 rounds to detect an increase in phages binding to the tumor cells but not to the PBL. After only 2 rounds of panning such an increase was seen with method 2, whereas method 1 and 3 showed an enrichment after 4 and 3 rounds, respectively. After subcloning into the expression vector pHOG21 a scFv ELISA was performed with 95 clones from round 3 of each method. Method 2 turned out to be best method considering not only the time point of enrichment (round 2) and but also the amount of scFv binders (39/95) as compared to method 1 (round 4, 11/94) and method 3 (round 3, 27/95).

### Comparison of methods 2 and 4:

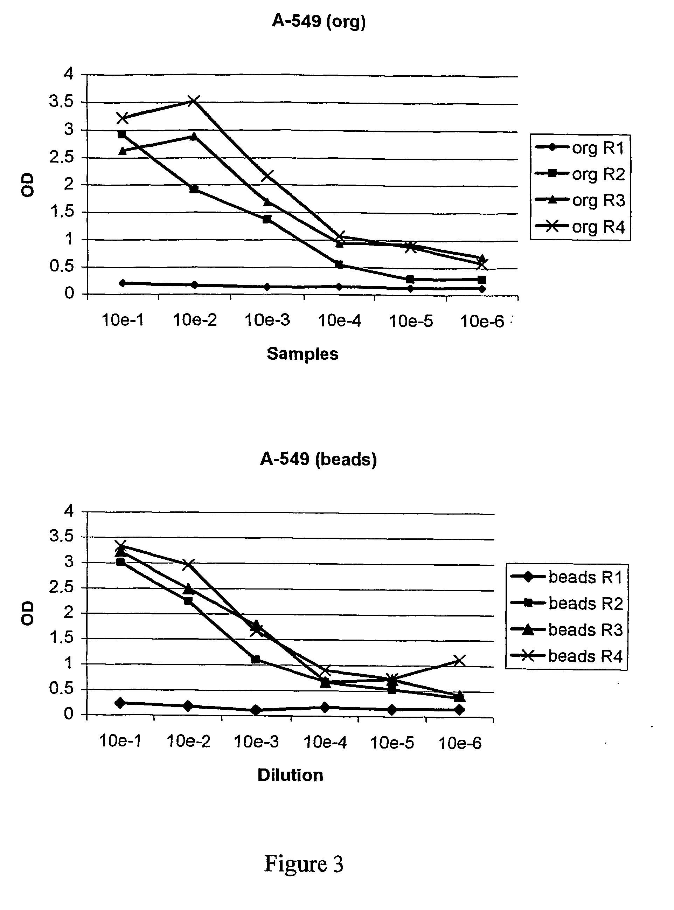

Results of the polyclonal phage ELISA revealed that after only 2 rounds of panning an increase in phages binding to the tumor cells was seen with both method 2 and 4. In contrast, method 2 showed an increased enrichment (curve is going up after diluting the phages 10⁻²) after the third and fourth round of panning when compared to method 4 (Figure 3).

Thus, method 2 (i.e. the method of the invention) shows significant advantages over prior art methods, especially in terms of being able to conduct fewer rounds of panning and identifying more positive clones.

### Example 3 - Successful panning projects using organic phase separation after 1 or 2 washes.

Two panning projects have successfully used the combination of 1 or 2 washes and organic phase separation:

### 1) Breast carcinoma project

A breast carcinoma cell line (PM-1) has been used for panning with a phage scFv display library. Negative pre-panning was performed on fresh human PBL as described in the Materials and Methods section. In these experiments the effect of washing before organic phase centrifugation and its comparison with the classical method was investigated (see above).

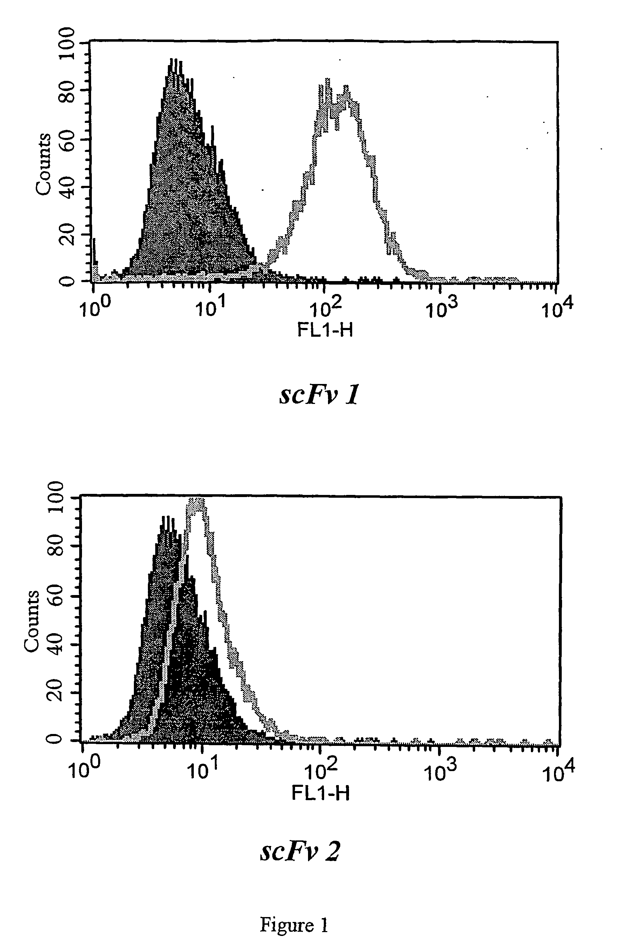

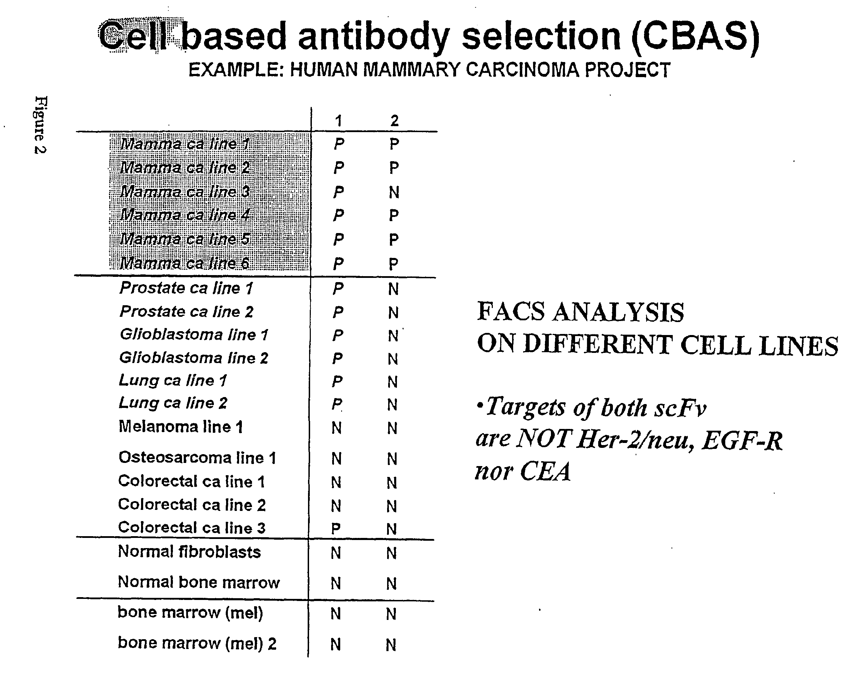

After one wash and organic phase centrifugation, single phages as well as scFv from round 3 of panning were tested for its specificity in cell ELISA and FACS measurements against PBL and the tumor cell line. Three unique and tumor cell line-specific clones were found (clones 1, 2 and 3). Testing single scFv expressions with the 8200 Cellular Detection System and Guava measurements, five more unique and tumor cell line-specific clones were found (data not shown). Closer investigation of the first three clones at the Norwegian Radium Hospital (DNR, Oslo, Norway) in FACS experiments showed that one scFv clone (1) binds primarily breast carcinoma cell lines, whereas another scFv clone (2) binds to tumor cell lines of different origin, e.g. prostate, glioblastoma, lung and colorectal tumour cell lines (Figures 1 and 2). Results have also shown that the target antigens for both these clones on tumour cells are not Her-2/neu, EGF-R nor CEA, thereby suggesting that they might recognise a novel tumour antigen. FACS and confocal microscopy data showed that in this case both scFv clone 1 and 2 bind to internalizing antigens.

Thus, it can be seen that the methods of the invention can be used to identify specific binding partners (in this case scFv antibody fragments) for molecules expressed on the surface of cells.

### 2) Lung carcinoma project

A lung carcinoma cell line (A-549) has been used for panning with a phage scFv display library. Negative pre-panning was performed on fresh human PBL and an endothelial cell line (HUVEC) as described in the Materials and Methods section. In these experiments a direct comparison was made between the method using 2 washings before organic phase centrifugation (method 2) and the method using lectin-coated magnetic beads for separation of free from cell-bound phages (method 4).

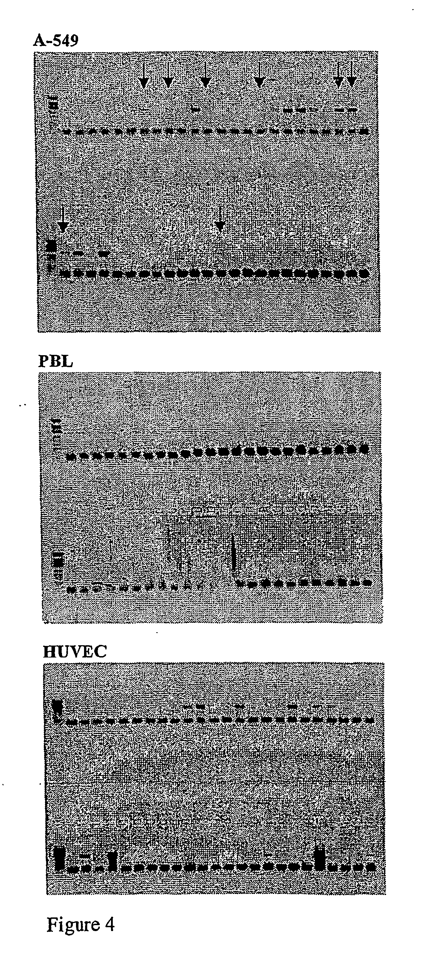

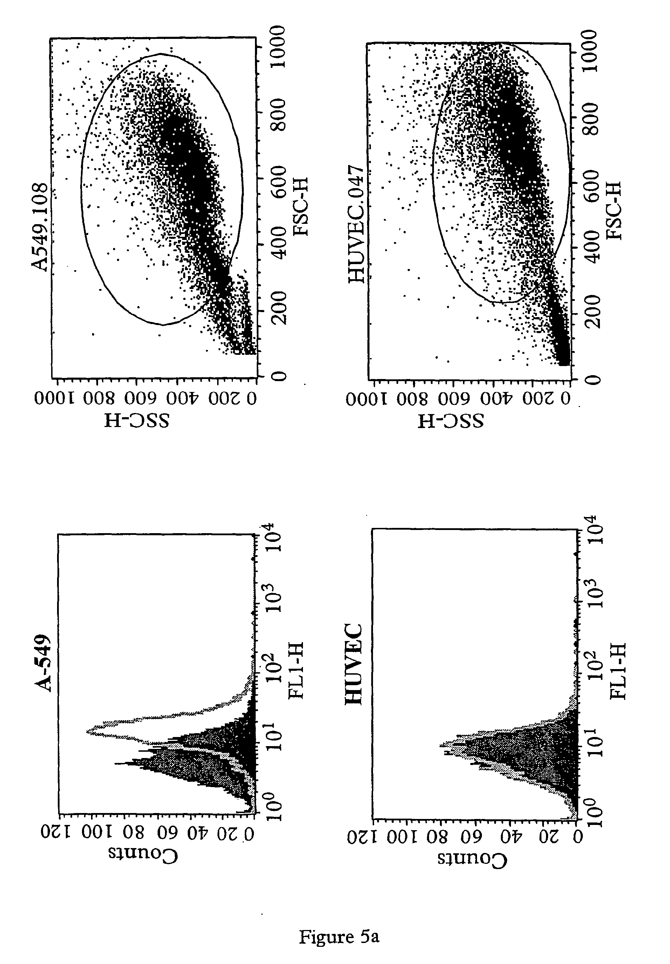

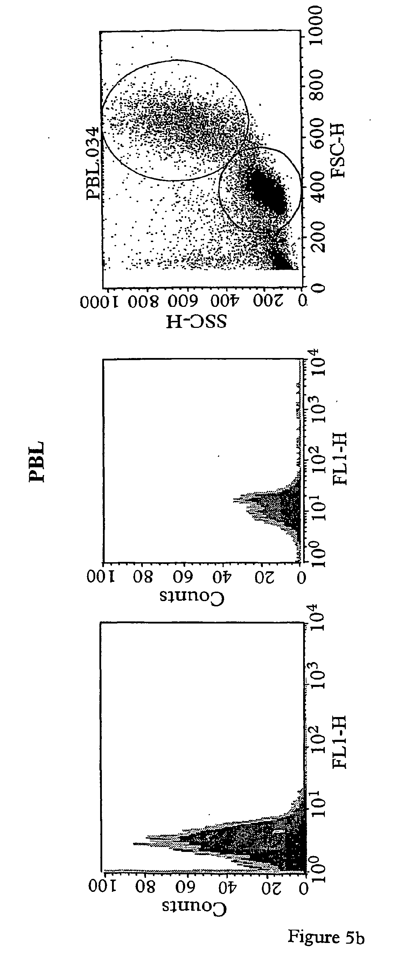

After two washes and organic phase centrifugation, scFv expressions from round 3 of panning were tested for its specificity in AffiSelect and cell ELISA against PBL, HUVEC and the tumor cell line A-549 (figure 4). As shown in the example of figure 4, eight scFv expression samples tested against the tumor cell line showed PCR-amplified beta-actin DNA, whereas no such product was seen with HUVEC and PBL. This shows that in this case 8 of the tested scFv samples were binding to the tumor cell line, but not to HUVEC or PBL. After confirming tumor cell line-specific binding by FACS (Figure 5 shows an exemplary clone) and Guava measurements (data not shown), ten unique and tumor cell line-specific clones were found.

Thus, it can again be seen that the methods of the invention can be used to identify specific binding partners (in this case scFv antibody fragments) for molecules expressed on the surface of cells.

### Example 4 - Testing different brands of PCR tubes

0.5 ml thin walled PCR tubes of different brands were tested for their stability during organic phase centrifugation and cutting after freezing.

0.5 ml thin-walled Eppendorf PCR tubes (#0030 124.502) and Axygen PCR-05-C (#321-05-051) were the best tubes tested. After freezing the organic phase, tubes had to be cut just above the V-shaped area to avoid splitting of the tube.

### Example 5 - Effect of organic phase on phage infectivity

10⁸ and 10¹¹ phages together with 100 and 200µl organic phase were used to infect 10 ml of E.Coli XL1-Blue (Stratagene) in log phase. Bacteria were incubated at 37°C for 15 min. at 60 rpm, then for 45 min at 200 rpm and plated at different concentrations on LB_{TAG} plates. After overnight incubation at 37°C the number of colonies was counted.

Working with low phage-titer (10⁸ compared to 10¹¹), the presence of organic phase had a negative effect (reduction of app. 50%) on the infectivity rate. At high phage titer, no such effect was seen.

As it can be seen from the results and discussion herein, with the described methods for screening antibody expression libraries with cells, screening for antibodies is not dependent on the availability of purified and/or recombinant antigen and even new cell type-specific antigens can be discovered. The combination of cellular panning using the methods of the invention in combination with cellular filter screening or other methods, such as AffiSelect or the 8200 Cellular Detection System (Applied Biosystems), thus provide a technology platform for high-throughput screening and selection of antibodies against cell surface antigens.

### Example 6 - Target identification of tumor specific clones from successful panning projects using organic phase separation after 1 or 2 washes.

Two scFv clones (clone 1 and 2, see Figures 1 and 2) from the breast carcinoma panning project using organic phase centrifugation after 1 wash were used for the identification of their target entities.

For the target identification a tumor cDNA phage display library was constructed from a positive cell line (SW900; highly expressing antigens to both scFv 1 and 2). This library was then used for panning on tubes, coated with scFv 1 and 2 respectively. After 3 rounds of panning, expressions of single tumor cDNA clones were tested by filterscreening for their binding to scFv 1 and 2 coated filters.

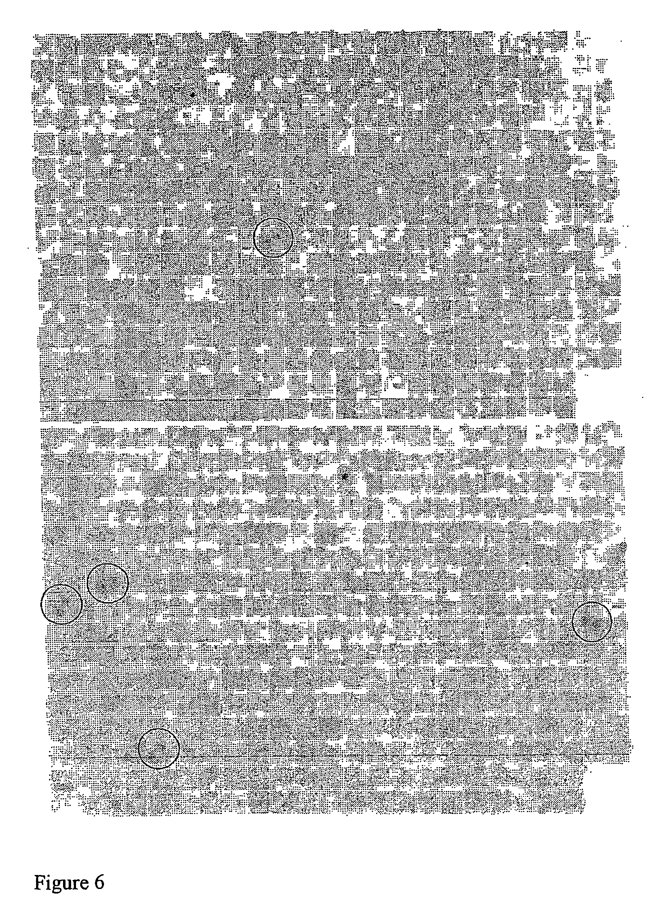

In case of scFv 2 antigen identification, 5 tumor cDNA clones were found that bind to the scFv 2-coated filter (Figure 6). Sequence analysis showed that all 5 clones correspond to the same nucleotide sequence.

In case ofscFv 1 antigen identification, 12 tumor cDNA clones showed binding to the scFv 1-coated filter (data not shown).

Binding of the tumor cDNA clones to the scFv was verified by ELISA on scFv coated plates. In addition, both scFvs 1 and 2 are now used in immune precipitation experiments, 1 and 2D PAGE, peptide extraction and mass spectrometry analysis.

Thus, it can be seen that the methods of the invention can further be used to identify target entities (in this case target antigens) which interact with the binding partners identified by the improved organic phase separation methods described herein.

## Claims

1. A method of screening a library of molecules to identify or select one or more members thereof which are candidate binding partners for one or more target entities comprising:
(a) contacting an expression library with one or more target entities;
(b) subjecting said target entities to at least one washing step;
(c) separating target entities which have become bound to one or more members of the expression library from unbound members of the expression library by separation through an organic phase, thereby separating candidate binding partners for said target entities from other library members,
wherein said expression library is a phage display library and wherein said target entity is a cell surface molecule or a molecule which is attached to a solid phase.

2. The method of claim 1 wherein said expression library is an antibody expression library.

3. The method of claim 2 wherein said antibody expression library comprises scFv antibodies.

4. The method of any one of claims 1-3 wherein said cell surface molecule is provided as a component of whole cells or as a membrane fraction of cells.

5. The method of any one of claims 1 to 4 wherein said target entity is an antigen.

6. The method of any one of claims 1 to 5 wherein said cells are transformed or transfected with nucleic acid encoding said target entity or are transformed or transfected with nucleic acids encoding a library of target entities, or are cells which overexpress said target entity.

7. The method of any one of claims 1 to 6 wherein said cells are eukaryotic cells.

8. The method of any one of claims 1 to 7 wherein said cells are associated with a disease state.

9. The method of any one of claims 1 to 8 wherein said cells are cancer cells.

10. The method of any one of claims 1 to 9 wherein said solid phase is a particulate solid phase.

11. The method of any one of claims 1 to 10 wherein more than one target entity is provided in step (a).

12. The method of any one of claims 1 to 11 wherein at least 2, at least 3, or at least 4 washing steps are carried out in step (b).

13. The method of any one of claims 1 to 11 wherein 1, 2, 3 or 4 washing steps are carried out in step (b).

14. The method of any one of claims 1 to 13 wherein said separation through an organic phase is carried out by centrifugation.

15. The method of any one of claims 1 to 14 wherein said method further comprises one or more pre-panning steps carried out before step (a) of the method, wherein said expression library is contacted with one or more non-relevant entities.

16. The method of any one of claims 1 to 15 wherein steps (a) to (c) are repeated one or more times.

17. The method of any one of claims 1 to 16 wherein steps (a) to (c) are repeated 1, 2 or 3 times.

18. The method of any one of claims 1 to 17 wherein said target entities which are bound to one or more members of the expression library are subjected to further analysis.

19. The method of claim 18 wherein said further analysis is a further analysis of the candidate binding partners and/or the target entities.

20. The method of any one of claims 1 to 19 further comprising a step wherein said candidate binding partners are detached, removed, eluted, or isolated from said target entities, or are expressed or produced in isolation from said target entities.

21. The method of claim 19 or claim 20 wherein said further analysis of the candidate binding partners comprises the steps of:
i) contacting one or more of said candidate binding partners in solution with one or more target entities;
ii) capturing target entities which have become bound to one or more of the candidate binding partners onto a solid phase; and
iii) detecting the presence of a target entity, thereby detecting the presence of one or more candidate binding partners for the target entity.

22. The method of claim 21 wherein said target entity is as defined in any one of the preceding claims.

23. The method of claim 21 or claim 22 wherein said candidate binding partners comprise a tag or marker to facilitate the capture step (ii).

24. The method of any one of claims 21 to 23 wherein said target entities contain or are associated with a reporter moiety to enable the detection of the target entities.

25. The method of claim 24 wherein said reporter moiety is a DNA molecule.

26. The method of claim 24 or claim 25 wherein said target entity is a cell surface molecule and said reporter moiety is a DNA molecule present in the cells on which the target entity is expressed.

27. The method of claim 25 or claim 26 wherein said DNA molecule is an endogenous housekeeping gene or is an exogenous reporter moiety.

28. The method of any one of claims 21 to 27 wherein said solid phase in step (ii) is magnetic and preferably particulate.

29. The method of any one of claims 21 to 28 wherein said capture step is facilitated by an interaction between a capture molecule on the solid phase with a tag or molecule associated with the candidate binding partner.

30. The method of any one of claims 21 to 29 wherein said detection step is carried out by detecting the presence of a reporter moiety on or associated with the target entity.

31. The method of claim 30 wherein the detection step is by PCR.

32. A method for isolating and/or identifying an unknown target entity comprising the steps as defined in any one of claims 1 to 31 and further comprising:
(d) isolating one or more expression library members which bind to said unknown target entity and
(e) using said library member to isolate and/or identify the target entity to which it binds, wherein said expression library is a phage display library and wherein said target entity is a cell surface molecule or a molecule which is attached to a solid phase.

33. A method of selecting, identifying and/or isolating a library member which is a specific binding partner for a target entity, or a method of selecting, identifying and/or isolating a target entity, from an expression library, said method comprising the steps as defined in any one of claims 1 to 31 to select molecules which display certain properties and optionally (e) identifying and/or isolating the relevant library member(s) which are specific binding partners for the target entity and optionally (f) using said library member to identify and/or isolate the target entity to which it binds.

34. The method of claim 32 or claim 33 wherein step (e) of claim 32 or step (f) of claim 33 comprises using said library member to screen a cDNA library prepared from cells on which the unknown target entity is expressed.

## Patentansprüche

1. Verfahren zum Screenen einer Bibliothek von Molekülen, um ein oder mehrere Mitglieder davon zu identifizieren oder auszuwählen, die Kandidatenbindungspartner für ein oder mehrere Zieleinheiten sind, das umfasst:
(a) Kontaktieren einer Expressionsbibliothek mit einer oder mehreren zieleinheiten;
(b) Unterziehen der Zieleinheiten mindestens einem Waschschritt;
(c) Trennen von Zieleinheiten, die an ein oder mehrere Mitglieder der Expressionsbibliothek gebunden wurden, von ungebundenen Mitgliedern der Expressionsbibliothek durch Trennung durch eine organische Phase, wodurch Kandidatenbindungspartner für die Zieleinheiten von anderen Bibliotheksmitgliedern getrennt werden,
wobei die Expressionsbibliothek eine Phagen-Displaybibliothek ist und wobei die Zieleinheit ein Zelloberflächenmolekül oder ein Molekül, das an eine feste Phase gebunden ist, ist.

2. Verfahren nach Anspruch 1, wobei die Expressionsbibliothek eine Antikörper-Expressionsbibliothek ist.

3. Verfahren nach Anspruch 2, wobei die Antikörper-Expressionsbibliothek scFv-Antikörper aufweist.

4. Verfahren nach einem der Ansprüche 1-3, wobei das Zelloberflächenmolekül als Komponente von ganzen Zellen oder als Membranfraktion von Zellen vorgesehen ist.

5. Verfahren nach einem der Ansprüche 1 bis 4, wobei die Zieleinheit ein Antigen ist.

6. Verfahren nach einem der Ansprüche 1 bis 5, wobei die Zellen mit einer Nukleinsäure transformiert oder transfiziert sind, die die Zieleinheit codiert, oder mit Nukleinsäuren transformiert oder transfiziert sind, die eine Bibliothek von Zieleinheiten codieren, oder Zellen sind, die die Zieleinheit überexprimieren.

7. Verfahren nach einem der Ansprüche 1 bis 6, wobei die Zellen eukaryotische Zellen sind.

8. Verfahren nach einem der Ansprüche 1 bis 7, wobei die Zellen zu einem Krankheitszustand gehören.

9. Verfahren nach einem der Ansprüche 1 bis 8, wobei die Zellen Krebszellen sind.

10. Verfahren nach einem der Ansprüche 1 bis 9, wobei die feste Phase eine feste Partikelphase ist.

11. Verfahren nach einem der Ansprüche 1 bis 10, wobei mehr als eine Zieleinheit in Schritt (a) vorgesehen wird.

12. Verfahren nach einem der Ansprüche 1 bis 11, wobei mindestens 2, mindestens 3 oder mindestens 4 Waschschritte in Schritt (b) ausgeführt werden.

13. Verfahren nach einem der Ansprüche 1 bis 11, wobei 1, 2, 3 oder 4 Waschschritte in Schritt (b) ausgeführt werden.

14. Verfahren nach einem der Ansprüche 1 bis 13, wobei die Trennung durch eine organische Phase durch Zentrifugation ausgeführt wird.

15. Verfahren nach einem der Ansprüche 1 bis 14, wobei das Verfahren weiterhin einen oder mehrere Vor-Panning-Schritte umfasst, die vor dem Schritt (a) des Verfahrens ausgeführt werden, wobei die Expressionsbibliothek mit einer oder mehreren nicht relevanten Einheiten in Kontakt gebracht wird.

16. Verfahren nach einem der Ansprüche 1 bis 15, wobei die Schritte (a) bis (c) einmal oder mehrmals wiederholt werden.

17. Verfahren nach einem der Ansprüche 1 bis 16, wobei die Schritte (a) bis (c) 1-, 2- oder 3-mal wiederholt werden.

18. Verfahren nach einem der Ansprüche 1 bis 17, wobei die Zieleinheiten, die an ein oder mehrere Mitglieder der Expressionsbibliothek gebunden werden, einer weiteren Analyse unterzogen werden.

19. Verfahren nach Anspruch 18, wobei die weitere Analyse eine weitere Analyse der Kandidatenbindungspartner und/oder der Zieleinheiten ist.

20. Verfahren nach einem der Ansprüche 1 bis 19, das weiterhin einen Schritt umfasst, in dem die Kandidatenbindungspartner von den Zieleinheiten gelöst, entfernt, eluiert oder isoliert werden oder von den Zieleinheiten isoliert exprimiert oder erzeugt werden.

21. Verfahren nach Anspruch 19 oder Anspruch 20, wobei die weitere Analyse der Kandidatenbindungspartner die Schritte umfasst:
i) Kontaktieren von einem oder mehreren der Kandidatenbindungspartner in Lösung mit einer oder mehreren Zieleinheiten;
ii) Erfassen von Zieleinheiten, die an einen oder mehrere der Kandidatenbindungspartner gebunden wurden, an einer festen Phase; und
iii) Detektieren der Anwesenheit einer Zieleinheit, wodurch die Anwesenheit von einem oder mehreren Kandidatenbindungspartnern für die Zieleinheit detektiert wird.

22. Verfahren nach Anspruch 21, wobei die Zieleinheit wie in einem der vorangehenden Ansprüche definiert ist.

23. Verfahren nach Anspruch 21 oder Anspruch 22, wobei die Kandidatenbindungspartner eine Markierung oder einen Marker aufweisen, um den Erfassungsschritt (ii) zu erleichtern.

24. Verfahren nach einem der Ansprüche 21 bis 23, wobei die Zieleinheiten einen Reporteranteil enthalten oder einem solchen zugeordnet sind, um die Detektion der Zieleinheiten zu ermöglichen.

25. Verfahren nach Anspruch 24, wobei der Reporteranteil ein DNA-Molekül ist.

26. Verfahren nach Anspruch 24 oder Anspruch 25, wobei die Zieleinheit ein Zelloberflächenmolekül ist und der Reporteranteil ein DNA-Molekül ist, das in den Zellen vorhanden ist, an denen die Zieleinheit exprimiert ist.

27. Verfahren nach Anspruch 25 oder Anspruch 26, wobei das DNA-Molekül ein endogenes konstitutives Gen ist oder ein exogener Reporteranteil ist.

28. Verfahren nach einem der Ansprüche 21 bis 27, wobei die feste Phase in Schritt (ii) magnetisch und vorzugsweise partikelförmig ist.

29. Verfahren nach einem der Ansprüche 21 bis 28, wobei der Erfassungsschritt durch eine Wechselwirkung zwischen einem Erfassungsmolekül an der festen Phase und einer Markierung oder einem Molekül, das dem Kandidatenbindungspartner zugeordnet ist, erleichtert wird.

30. Verfahren nach einem der Ansprüche 21 bis 29, wobei der Detektionsschritt durch Detektieren der Anwesenheit eines Reporteranteils an der Zieleinheit oder eines der Zieleinheit zugeordneten Reporteranteils ausgeführt wird.

31. Verfahren nach Anspruch 30, wobei der Detektionsschritt durch PCR stattfindet.

32. Verfahren zum Isolieren und/oder Identifizieren einer unbekannten Zieleinheit mit den Schritten, wie in einem der Ansprüche 1 bis 31 definiert, und weiterhin umfassend:
(d) Isolieren von einem oder mehreren Expressionsbibliotheks-Mitgliedern, die an die unbekannte Zieleinheit binden, und
(e) Verwenden des Bibliotheksmitglieds, um die Zieleinheit, an die es bindet, zu isolieren und/oder zu identifizieren, wobei die Expressionsbibliothek eine Phagen-Displaybibliothek ist und wobei die Zieleinheit ein Zelloberflächenmolekül oder ein Molekül ist, das an eine feste Phase gebunden ist.

33. Verfahren zum Auswählen, Identifizieren und/oder Isolieren eines Bibliotheksmitgliedes, das ein spezifischer Bindungspartner für eine Zieleinheit ist, oder Verfahren zum Auswählen, Identifizieren und/oder Isolieren einer Zieleinheit von einer Expressionsbibliothek, wobei das Verfahren die Schritte, wie in einem der Ansprüche 1 bis 31 definiert, umfasst, um Moleküle, die bestimmte Eigenschaften zeigen, auszuwählen, und gegebenenfalls (e) Identifizieren und/oder Isolieren des (der) relevanten Bibliotheksmitgliedes (Bibliotheksmitglieder), die spezifische Bindungspartner für die Zieleinheit sind, und gegebenenfalls (f) Verwenden des Bibliotheksmitgliedes, um die Zieleinheit, an die es bindet, zu identifizieren und/oder zu isolieren.

34. Verfahren nach Anspruch 32 oder Anspruch 33, wobei Schritt (e) von Anspruch 32 oder Schritt (f) von Anspruch 33 die Verwendung des Bibliotheksmitgliedes zum Screenen einer cDNA-Bibliothek umfasst, die aus Zellen vorbereitet ist, an denen die unbekannte Zieleinheit exprimiert ist.

## Revendications

1. Procédé de criblage d'une banque de molécules pour identifier ou sélectionner un ou plusieurs éléments de celle-ci qui sont des partenaires de liaison candidats pour une ou plusieurs entités cibles comprenant :
(a) la mise en contact d'une banque d'expression avec une ou plusieurs entités cibles ;
(b) la soumission desdites entités cibles à au moins une étape de lavage ;
(c) la séparation d'entités cibles qui se sont liées à un ou plusieurs éléments de la banque d'expression d'éléments non liés de la banque d'expression par séparation par l'intermédiaire d'une phase organique, séparant ainsi des partenaires de liaison candidats pour lesdites entités cibles d'autres éléments de la banque ;
dans lequel ladite banque d'expression est une banque d'exposition sur phage et dans lequel ladite entité cible est une molécule de surface cellulaire ou une molécule qui est fixée à une phase solide.

2. Procédé selon la revendication 1 dans lequel ladite banque d'expression est une banque d'expression d'anticorps.

3. Procédé selon la revendication 2 dans lequel ladite banque d'expression d'anticorps comprend des anticorps scFv.

4. Procédé selon l'une quelconque des revendications 1 à 3 dans lequel ladite molécule de surface cellulaire est prévue comme un composant de cellules entières ou comme une fraction membranaire de cellules.

5. Procédé selon l'une quelconque des revendications 1 à 4 dans lequel ladite entité cible est un antigène.

6. Procédé selon l'une quelconque des revendications 1 à 5 dans lequel lesdites cellules sont transformées ou transfectées avec un acide nucléique codant pour ladite entité cible ou sont transformées ou transfectées avec des acides nucléiques codant pour une banque d'entités cibles, ou sont des cellules qui surexpriment ladite entité cible.

7. Procédé selon l'une quelconque des revendications 1 à 6 dans lequel lesdites cellules sont des cellules eucaryotes.

8. Procédé selon l'une quelconque des revendications 1 à 7 dans lequel lesdites cellules sont associées avec un état de maladie.

9. Procédé selon l'une quelconque des revendications 1 à 8 dans lequel lesdites cellules sont des cellules cancéreuses.

10. Procédé selon l'une quelconque des revendications 1 à 9 dans lequel ladite phase solide est une phase solide particulaire.

11. Procédé selon l'une quelconque des revendications 1 à 10 dans lequel plus d'une entité cible est prévue à l'étape (a).

12. Procédé selon l'une quelconque des revendications 1 à 11 dans lequel au moins 2, au moins 3, ou au moins 4 étapes de lavage sont mises en oeuvre à l'étape (b).

13. Procédé selon l'une quelconque des revendications 1 à 11 dans lequel 1, 2, 3 ou 4 étapes de lavage sont mises en oeuvre à l'étape (b).

14. Procédé selon l'une quelconque des revendications 1 à 13 dans lequel ladite séparation par l'intermédiaire d'une phase organique est mise en oeuvre par centrifugation.

15. Procédé selon l'une quelconque des revendications 1 à 14 dans lequel ledit procédé comprend en outre une ou plusieurs étapes de pré-adhérence sur plastique mises en oeuvre avant l'étape (a) du procédé, dans lequel ladite banque d'expression est mise en contact avec une ou plusieurs entités non pertinentes.

16. Procédé selon l'une quelconque des revendications 1 à 15 dans lequel les étapes (a) à (c) sont répétées une ou plusieurs fois.

17. Procédé selon l'une quelconque des revendications 1 à 16 dans lequel les étapes (a) à (c) sont répétées 1, 2 ou 3 fois.

18. Procédé selon l'une quelconque des revendications 1 à 17 dans lequel lesdites entités cibles qui sont liées à un ou plusieurs éléments de la banque d'expression sont soumises à analyse plus poussée.

19. Procédé selon la revendication 18 dans lequel ladite analyse plus poussée est une analyse plus poussée des partenaires de liaison candidats et/ou des entités cibles.

20. Procédé selon l'une quelconque des revendications 1 à 19 comprenant en outre une étape dans laquelle lesdits partenaires de liaison candidats sont détachés, enlevés, élués, ou isolés desdites entités cibles, ou sont exprimés ou produits de façon isolée desdites entités cibles.

21. Procédé selon la revendication 19 ou la revendication 20 dans lequel ladite analyse plus poussée des partenaires de liaison candidats comprend les étapes de :
i) mise en contact d'un ou plusieurs desdits partenaires de liaison candidats en solution avec une ou plusieurs entités cibles ;
ii) capture des entités cibles qui se sont liées à un ou plusieurs des partenaires de liaison candidats sur une phase solide ; et
iii) détection de la présence d'une entité cible, détectant ainsi la présence d'un ou plusieurs partenaires de liaison candidats pour l'entité cible.

22. Procédé selon la revendication 21 dans lequel ladite entité cible est telle que définie dans l'une quelconque des revendications précédentes.

23. Procédé selon la revendication 21 ou la revendication 22 dans lequel lesdits partenaires de liaison candidats comprennent une étiquette ou un marqueur pour faciliter l'étape de capture (ii).

24. Procédé selon l'une quelconque des revendications 21 à 23 dans lequel lesdites entités cibles contiennent ou sont associées avec un groupe caractéristique rapporteur pour permettre la détection des entités cibles.

25. Procédé selon la revendication 24 dans lequel ledit groupe caractéristique rapporteur est une molécule d'ADN.

26. Procédé selon la revendication 24 ou la revendication 25 dans lequel ladite entité cible est une molécule de surface cellulaire et ledit groupe caractéristique rapporteur est une molécule d'ADN présente dans les cellules sur lesquelles l'entité cible est exprimée.

27. Procédé selon la revendication 25 ou la revendication 26 dans lequel ladite molécule d'ADN est un gène domestique endogène ou est un groupe caractéristique rapporteur exogène.

28. Procédé selon l'une quelconque des revendications 21 à 27 dans lequel ladite phase solide à l'étape (ii) est magnétique et préférablement particulaire.

29. Procédé selon l'une quelconque des revendications 21 à 28 dans lequel ladite étape de capture est facilitée par une interaction entre une molécule de capture sur la phase solide avec une étiquette ou une molécule associée avec le partenaire de liaison candidat.

30. Procédé selon l'une quelconque des revendications 21 à 29 dans lequel ladite étape de détection est mise en oeuvre en détectant la présence d'un groupe caractéristique rapporteur sur ou associé avec l'entité cible.

31. Procédé selon la revendication 30 dans lequel l'étape de détection est mise en oeuvre par PCR.

32. Procédé d'isolation et/ou d'identification d'une entité cible inconnue comprenant les étapes telles que définies dans l'une quelconque des revendications 1 à 31 et comprenant en outre :
(d) l'isolation d'un ou plusieurs membres de la banque d'expression qui se lient à ladite entité cible inconnue et
(e) l'utilisation dudit élément de la banque pour isoler et/ou identifier l'entité cible à laquelle il se lie, dans lequel ladite banque d'expression est une banque d'exposition sur phage et dans lequel ladite entité cible est une molécule de surface cellulaire ou une molécule qui est fixée à une phase solide.

33. Procédé de sélection, d'identification et/ou d'isolation d'un élément d'une banque qui est un partenaire de liaison spécifique pour une entité cible, ou procédé de sélection, d'identification et/ou d'isolation d'une entité cible, à partir d'une banque d'expression, ledit procédé comprenant les étapes telles que définies dans l'une quelconque des revendications 1 à 31 pour sélectionner des molécules qui affichent certaines propriétés et facultativement (e) l'identification et/ou l'isolation du (des) membre(s) pertinent(s) de la banque qui sont des partenaires de liaison spécifiques pour l'entité cible et facultativement (f) l'utilisation dudit élément de la banque pour identifier et/ou isoler l'entité cible à laquelle il se lie.

34. Procédé selon la revendication 32 ou la revendication 33 dans lequel l'étape (e) de la revendication 32 ou l'étape (f) de la revendication 33 comprend l'utilisation dudit élément de la banque pour cribler une banque d'ADNc préparée à partir de cellules sur lesquelles l'entité cible inconnue est exprimée.
